# EUROPEAN PATENT APPLICATION

(11) **EP 2 199 798 A1**
(43) Date of publication of application: **23.06.2010**
(21) Application number: 08172217.5
(22) Date of filing: 18.12.2008
(51) Int. Cl.: G01N 33/574

(54) **Diagnostic method and kit of circulating tumor cells**

(71) Applicant: UNIVERSITE LIBRE DE BRUXELLES, 1050 Brussels (BE)
(72) Inventor: Sotiriou, Christos, 1080 Bruxelles (BE); Ignatiadis, Michail, 54644 Thessaloniki (GR)
(74) Representative: pronovem

(57) **Abstract**

The present invention is related to a method and a diagnostic kit for an improved detection (diagnosis) of early breast cancer by combining screening mammography together with HER2-positive circulating tumor cells (CTCs) possibly present in a mammal subject (including a human patient).

## Description

### Field of the invention

The present invention is related to a method and a diagnostic kit for an improved detection (diagnosis) of early breast cancer by combining screening mammography together with HER2-positive circulating tumor cells (CTCs) possibly present in a mammal subject (including a human patient).

### Background of the invention

Breast cancer is the most common cancer in women in Western countries.

The presence of CTCs in the blood of a cancer patient is associated with a worse prognosis.

Veridex corp developed a test kit for the isolation of CTC in the peripheral blood of a patient to determine the prognosis of patients with metastatic breast, colorectal or prostate cancer at any time. The test offers an objective, quantitative, real-time reading of tumor information so that oncologists can provide optimum care for their patients. This test is based on an isolation of cells via their epithelial proteins, such as Epithelial cell adhesion molecule (EPCAM) and/or cytokeratins 8, 18 or 19. In brief, CTC are bound and enriched on beads derivatized with anti EPCAM antibodies, followed by a labelling using anti CD45 (negative control; leukocyte marker), a labelling for the nuclei (positive control: all cells) and cytokeratins (CTC). However, this test is not yet accepted for diagnostic use: its sensitivity remains to be demonstrated in clinical diagnostics.

Another test sold by Veridex allows HER2 tumor-phenotyping. This test also is for research-use only. More particularly, this test may lack of sensitivity and of quantitative measurements.

These tests are sold to monitor a woman diagnosed with breast cancer in order to follow if said woman has circulating tumor cells and to follow any change in the number of CTC, however there is no indication on the use of these tests for women not previously diagnosed as having a breast cancer, or who are suspected of having a breast cancer and/or who are at higher risk for developing breast cancer like a women with a pre-invasive cancer lesion or an abnormal mammogram.

Rack et al. launched a trial evidencing prognostic significance of CTCs in early breast cancer (SUCCESS trial), >1 CTC/23 ml of blood was detected by the CellSearch® System in 9.5% and 8.7% of 1500 node-positive and high risk node-negative early breast cancer patients before and after adjuvant chemotherapy, respectively. After a 12-month median follow up, detection of >1 CTC/23 ml after but not before adjuvant chemotherapy was associated with shorter disease-free and overall survival (Rack B. et al, Proc. Am.Soc.Clin.Oncol. vol 26: abstr 503 2008). However, the CTC positivity rate was low (<10%).

### Aims of the invention

The present invention aims to provide new detection method and tools that do not present the drawbacks of the state of the art.

The present invention aims to provide an improved diagnostic of the HER2 state of (putative) circulating tumor cells in a (woman) patient.

### Summary of the invention

The present invention discloses method and tools presenting (compared to the methods and tools of the state of the art) an improved sensitivity or specificity of an hyperproliferative disorder (cancer) detection, in (woman) patients considered as free from (not affected by) breast cancer or suffering from early breast cancer, possibly combined with standard imaging methods and devices, such as mammography or magnetic resonance imaging (MRI).

More precisely, the method and tools of the present invention are related to a diagnostic method and kit of the HER2 state of (putative) circulating tumor cells in breast cancer (woman) patients.

The method and kit of the present invention are applied to biological samples obtained from patients not previously diagnosed as having a breast cancer or a predisposition to develop a breast cancer.

The method and kit of the present invention are preferably applied to biological samples obtained from patients who are suspected of having a breast cancer.

The method and kit of the present invention are preferably applied to biological samples obtained from patients who are at higher risk for developing breast cancer, like patients with a pre-invasive (cancer) lesion or an abnormal mammogram.

Alternatively, the diagnostic of the HER2 state of putative circulating tumor cells in a (woman) patient is done upon biological samples obtained from patients suffering from early breast cancer.

The diagnostic of the HER2 state of putative circulating tumor cells in a (woman) patient may also be done upon biological samples obtained from patients suffering from metastatic breast cancer.

The present invention relates to a cancer diagnostic method which comprises the step of:
- a) submitting a patient blood sample to an enrichment of putative circulating tumor cells;
- b) labelling the retained cells of step a)
   for one, two, three or four epithelial marker, preferably cytokeratin 8 and/or cytokeratin 18 and/or cytokeratin 19 and/or mammaglobin; and for HER2 state;
- c) deducing and possibly quantifying a presence of circulating tumor cells in the sample, via a positive signal for epithelial marker detection and
- d) establishing an HER2 state of circulating tumor cells in the said sample.

The method of the invention advantageously comprises one or more enrichment step(s) of epithelial cells that may be present in a blood sample of a patient.

Preferably, recognition of this membrane marker is done by the use of an antibody or its specific hypervariable fragment both being directed against this marker of a membrane-marker of epithelial cells, said marker being preferably EpCAM.

Alternatively, the enrichment step of epithelial cells (in a blood sample) is based on centrifugation, preferably using Ficoll gradient.

Another preferred enrichment step consist on the use of cell filters (in a blood sample) able to retain epithelial cells, but not blood cells.

Advantageously, two enrichment methods of CTC are performed (in parallel) in the method of the invention.

The method of the invention advantageously further comprises the step of sorting non-epithelial cells based on a recognition of the membrane-marker not expressed by epithelial cells, preferably the extracellular part of a protein, being preferably CD45.

The method of the invention optionally comprises the step of labelling epithelial proteins and/or mRNA expressed in breast tissue, being preferably mammaglobins.

The method of the invention advantageously comprises the step of labelling epithelial proteins and/or mRNA expressed in epithelial tissue, these proteins and/or mRNA being preferably cytokeratin 8, 18 and/or 19.

Advantageously, these cytokeratin 8, 18 and/or 19 are labelled by an (one) antibody (a specific hypervariable fragment thereof or a mixture of antibodies) that specifically recognizes (that is(are) directed against) these three cytokeratin 8, 18 and 19 proteins.

Alternatively, an (one) antibody specific for a (one) protein selected from the group consisting of cytokeratin 8, cytokeratin 18 and cytokeratin 19 is used in the method and kit of the invention.

Alternatively, a specific detection of both cytokeratin 8 and cytokeratin 18 is performed.

Alternatively, a specific detection of both cytokeratin 8 and cytokeratin 19 is performed.

Alternatively, a specific detection of both cytokeratin 18 and cytokeratin 19 is performed.

Alternatively, a specific detection of both cytokeratin 8, cytokeratin 18 and cytokeratin 19 is performed.

Alternatively cytokeratin 8 and/or cytokeratin 18 and/or cytokeratin 19 mRNA and/or mammaglobin is identified and/or quantified using PCR.

The present invention concerns a cancer diagnostic and possibly monitoring method (possibly combined with standard imaging methods such as mammography or magnetic resonance imaging (MRI)) and comprising the step of:
- a) submitting a biological sample such as a fluid (preferably a blood sample) from a human (woman) patient, to an enrichment of putative circulating tumor cells, preferably upon anti-EPCAM antibodies (or specific hypervariable portion thereof) retention,
- b) labelling the obtained (retained) cells from step a) with non-epithelial markers, being preferably anti-CD45 antibodies (or specific hyper variable fragments thereof),
- c) labelling the obtained (retained) cells with epithelial markers, being preferably anti-cytokeratin (8, 18 or 19) antibodies (or a specific hyper variable fragment thereof),
- d) quantifying Her2 content, preferably via the labelling with of HER2 protein anti-HER2 antibodies (or specific hyper variable fragments thereof)
   and/or with Her2 mRNA content
   and/or with Her2 DNA content and/or with Her2 mRNA-related sequence (micro-RNA).
- e) deducing and possibly quantifying the presence of circulating tumor cells, via a positive signal for cytokeratin detection and a negative signal for CD45 detection,
- f) establishing the HER2 state (profile or level) of circulating tumor cells present in the sample.

The present invention concerns a cancer diagnostic and possibly monitoring method (possibly combined with standard imaging methods such as mammography or magnetic resonance imaging (MRI)) and comprising the step of:
- a) submitting a biological sample such as a fluid (preferably a blood sample) from a human (woman) patient, to an enrichment of putative circulating tumor cells, preferably upon centrifugation in Ficoll or through filtering,
- b) optionally labelling the obtained (retained) cells from step a) with non-epithelial markers, being preferably anti-CD45 antibodies (or specific hyper variable fragments thereof),
- c) measuring the obtained (retained) cells with epithelial markers, being preferably cytokeratin (8, 18 or 19) mRNA content and/or mammaglobin mRNA content,
- d) quantifying Her2 content, preferably via the labelling with of HER2 protein anti-HER2 antibodies (or specific hyper variable fragments thereof)
   and/or with Her2 mRNA content
   and/or with Her2 DNA content and/or with Her2 mRNA-related sequence (micro-RNA).
- e) deducing and possibly quantifying the presence of circulating tumor cells, via a positive signal for cytokeratin detection and a negative signal for CD45 detection,
- f) establishing the HER2 state (profile or level) of circulating tumor cells present in the sample.

The present invention concerns a cancer diagnostic and possibly monitoring method (possibly combined with standard imaging methods such as mammography or magnetic resonance imaging (MRI)) and comprising the step of:
- a) submitting a biological sample such as a fluid (preferably a blood sample) from a human (woman) patient, to an enrichment of putative circulating tumor cells;
- b) labelling (preferably a first part of) the obtained (retained) cells with epithelial markers, being preferably anti-cytokeratin (8, 18 or 19) antibodies (or a specific hyper variable fragment thereof),
- c) deducing and possibly quantifying the presence of circulating tumor cells(preferably in the first part of the said biological sample), via a positive signal for cytokeratin detection and a negative signal for CD45 detection,
- d) quantifying Her2 content of the cells of step c), preferably via the labelling with of HER2 protein with anti-HER2 antibodies (or specific hyper variable fragments thereof)
- e) submitting a biological sample such as a fluid (preferably a blood sample) from a human (woman) patient to an enrichment of putative circulating tumor cells,
- f) measuring the obtained (retained) cells (in steps a and/or e) with epithelial markers, being preferably cytokeratin (8, 18 or 19) mRNA content and/or mammaglobin mRNA content,
- g) deducing and possibly quantifying the presence of circulating tumor cells, via a positive signal for cytokeratin (8, 18, 19) or mammaglobin detection,
- h) quantifying Her2 content, preferably via the measurement of Her2 mRNA content;
- i) establishing the HER2 state (profile or level) for every circulating tumor cell identified in steps b-c or in steps f-g and possibly deducing the presence of Her2 positive CTC.

Advantageously, the said method further comprises a step of labelling the obtained (retained) cells from step a) with non-epithelial markers, being preferably anti-CD45 antibodies (or specific hyper variable fragments thereof),

The present diagnostic method may also be applied to biological samples obtained from women presenting pre-invasive lesions (in breast cancer) like hyperplasias or ductal or lobular carcinoma in situ or for women at higher risk of developing breast cancer, possibly based on the gail model (Gail MH, Brinton LA, Byar DP, Corle DK, Green SB, Shairer C, Mulvihill JJ: Projecting individualized probabilities of developing breast cancer for white females who are being examined annually. J Natl Cancer Inst 81(24):1879-86, 1989).

The present diagnostic method may also be applied to a patient (woman) that is not presently diagnosed as having a cancer.

Alternatively, the diagnostic method of the invention comprises the steps of:
- a) submitting a biological sample preferably a fluid (blood sample) to an enrichment of putative circulating tumor cells, preferably on anti-EPCAM antibodies (or specific hyper variable fragments thereof) retention, and preferably upon absence of binding to anti CD45 antibodies (or specific hyper variable fragments thereof);
- b) quantifying HER2 protein expression via the labelling with anti-HER2 antibodies (or specific hyper variable fragments thereof) for every circulating tumor cell enriched and
- c) deducing whether these circulating tumor cells present HER2+ characteristics (HER2+ state).

Alternatively, the diagnostic method of the invention comprises the steps of:
- submitting a biological fluid (blood sample) to an enrichment of putative circulating tumor cells, preferably on anti-EPCAM antibodies (or specific hyper variable fragments thereof) retention,
- preferably label and/or sort the said enriched cells with anti CD45 antibodies (or specific hyper variable fragments thereof);
- quantifying mRNA and/or gene amplification of HER2 for every enriched putative circulating tumor cell and
- deducing whether these putative circulating tumor cells present HER2+ characteristics (HER2+ state).

The present invention is also related to the tools for performing the essential steps of the method of the invention, especially related to a detection and possibly monitoring kit that comprises anti-EPCAM and anti-HER2 antibodies (or specific hyper variable portions thereof), and possibly anti-CD45 and/or anti-cytokeratin (8, 18 or 19) and/or mammaglobin antibodies (or specific hyper variable fragments thereof).

The present invention is related to a cancer diagnostic kit, which comprises an insoluble solid support, such as beads coupled with (grafted with or fixed to) anti-EpCAM antibodies or specific hyper variable fragments thereof
and anti-HER2 antibodies or specific hyper variable fragments thereof and/or means (primers and possibly detection probe) for Her2 mRNA quantification.

The cancer diagnostic kit of the invention may further comprise anti-CD45 antibodies and antibodies recognising at least one cytokeratin selected from the group consisting of cytokeratin 8, cytokeratin 18 and cytokeratin 19.

### Detailed description of the invention

Her2 (neu; also known as ErbB-2; Seq. ID. NOs. 11 and 12) stands for Human Epidermal growth factor 2 and is a protein giving higher aggressiveness in breast cancers. ErbB 2 is a receptor tyrosine kinase of the ErbB 2 family. It is closely related in structure to the epidermal growth factor receptor. ErbB 2 oncoprotein is detectable in a proportion of breast and other adenocarconomas, as well as transitional cell carcinomas. In the case of breast cancer, expression determined by immunohistochemistry has been shown to be associated with poor prognosis. The sequence of human Her2 protein, mRNA, gene and RNA-related sequences may be alternatively found using available databases.

This protein has no ligand binding domain of its own and therefore cannot bind growth factors. However, it does bind tightly to other ligand-bound EGF receptor family members to form a heterodimer, stabilizing ligand binding and enhancing kinase-mediated activation of downstream signalling pathways.

Allelic variations at amino acid positions 654 and 655 of isoform a (positions 624 and 625 of isoform b) have been reported.

Amplification and/or overexpression of this gene has been reported in numerous cancers, including breast and ovarian tumors.

Alternative splicing results in several additional transcript variants, some encoding different isoforms. All variants of Her2 are encompassed in the present invention.

CD45 (Seq. ID. NOs. 7 and 8) was originally called leukocyte common antigen. CD45 is a family of single chain transmembraneous glycoproteins consisting of at least four isoforms (220, 205, 190, 180 kDa) which share a common large intracellular domain. Their extracellular domains are heavily glycosylated. The different isoforms are produced by alternative messenger RNA splicing of three exons of a single gene on chromosome 1. CD45 is expressed on cells of the human hematopoietic lineage (including hematopoietic stem cells) with the exception of mature red cells. CD45 is not detected on differentiated cells of other tissues. Antibodies recognising a common epitope on all of the isoforms are termed CD45 antibodies. However, every variuants are encompassed in the present invention.

Cytokeratins are intermediate filament keratins found in the intracytoplasmic cytoskeleton of epithelial tissue.
The present patent application refers to the use of the mRNA and/or protein sequence of human cytokeratin 8 (SEQ. ID. Nos. 1 and 2) that may alternatively be found using databases
and/or to the use of mRNA and/or protein sequence of human cytokeratin 18 (Seq. ID. Nos. 3 and 4) that may alternatively be found using databases and/or
to the use of mRNA and/or protein sequence of human cytokeratin 19 (Seq. ID. Nos 5 and 6) that may alternatively be found using databases.

Epithelial Cell Adhesion Molecule (EpCAM; SEQ. ID. NOs. 9 and 10) is a 40kD cell surface antigen. This antigen has been identified independently by a number of groups, and has been known by a variety of names. Several monoclonal antibodies have been raised against EpCAM. EpCAM is a Type 1 transmembrane glycoprotein. It is expressed on the basolateral membrane of cells by the majority of epithelial tissues, with the exception of adult squamous epithelium and some specific epithelial cell types including hepatocytes and gastric epithelial cells.

EpCAM expression has been reported to be a possible marker of early malignancy, with expression being increased in tumour cells, and de novo expression being seen in dysplastic squamous epithelium. EPCAM is a cell surface molecule.

EPCAM accession number is NM_002354. Its Entrez gene id is 4072.

The present invention will be explained in more details in the following non-limiting example.

The skilled person may develop alternative without being outside the scope of the present invention. More particularly, the skilled person may find and/or use and/or develop other means to enrich circulating tumor cells, to label them and to assess HER2 state in these cells.

This can be done using antibodies, fragments of antibodies, other protein or peptides having an affinity for circulating tumor cells, or any ligand able to enrich and/or bind and/or label circulating tumor cells.

Her2 expression state can be determined by evidencing the translated protein (e.g. using specific antibodies or hyper variable fragments of antibodies), by measuring its mRNA content,
the content of RNA-related sequences (e.g. micro-RNA),
or quantification of gene copies number.

Her2 expression state is concluded as positive if at least one, preferably at least two, more preferably at least three, or the four of the test selected from the list consisting of quantification of the HER2 translated protein, of the Her2 mRNA content, of Her2 RNA-related sequences (e.g. micro-RNA) or of Her2 gene duplication is/are positive.

Her2 expression state is advantageously determined by the combined quantification of the translated HER2 protein and of the Her2 mRNA content.

Alternatively, Her2 expression state is determined by the combined quantification of the translated HER2 protein and of Her2 gene duplication.

Preferably, Her2 state of CTC is determined by the combined quantification of the HER2 protein and of the Her2 DNA by FISH (Fluorescence after in situ hybridization) in the same CTC.

Her2 state of CTC may alternatively be obtained by quantification of mRNA sequences (mRNA transcripts) and of the microRNA level of CTC.

Her2 state of CTC may be obtained by quantification of the HER2 protein and of the microRNA level of CTC.

By a patient, it is meant every a person susceptible to have and/or to develop a breast cancer.

By an insoluble solid support, it is meant every surface that may be grafted with (fixed by) antibodies.

Beads are an example of insoluble solid support.

The preferred beads present in the kit according to the invention are sedimentable.

### Example

CTCs have been detected using the CellSearch® System in early and metastatic breast cancer (BC). In this study, the inventors first aimed to analyze CTCs for HER2 expression in different stages of BC progression from ductal/lobular carcinoma in situ (DCIS/LCIS) to early and metastatic BC. This test was also applied to healthy woman as negative control and possible detection of non-diagnosed breast cancer.

The skilled person may use and/or develop other tests to isolate CTC, and to evaluate their Her2 state.

### Patients and Methods:

20 ml of peripheral blood per woman was analyzed from healthy women, DCIS/LCIS or early BC patients, whereas 7.5 ml of blood was analyzed from metastatic BC patients. The presence of CTCs as well as HER2 expression was assessed with the CellSearch® System (Veridex, USA). After immunomagnetic enrichment with an anti-Epcam-antibody, cells were labeled with anti-cytokeratin (8,18,19), anti-HER2 and anti-CD45 antibodies.

CTCs were defined as Cytokeratin-positive/CD45-negative cells, whereas HER2+ CTCs were defined as Cytokeratin-positive/CD45-negative/HER2-positive cells.

### Results:

The presence of CTCs and HER2+ CTCs in healthy women, DCIS/LCIS, early and metastatic BC is depicted in table 1.

**Table 1: identification and Her2 characterization of circulating tumor cells in healthy woman, precancerous and cancerous patients.**

| | Women studied | Women with CTCs | | | Women with HER2+ CTCs |
|---|---|---|---|---|---|
| | | 1 CTC | 2CTCs | >2CTCs | ≥1 CTC |
| Healthy women | 47 | 7 | 4 | 0 | 0 |
| DCIS/LCIS | 40 | 8 | 2 | 1 | 8 |
| Early | 37 | 5 | 2 | 5 | 11 |
| Metastatic | 21 | 3 | 1 | 8 | 7 |

Her2 state can alternatively be measured by PCR, for instance using nested PCR with
Forward primer1 (SEQ.ID.NO.13): TCC TCC TCG CCC TCT TGC ;
Forward primer2 (SEQ.ID.NO.14): AGC CGC GAG CAC CCA AGT;
Reverse primer1: (SEQ.ID.NO.15)GCG GGT CTC CAT TGT CTA and
Reverse primer2: (SEQ.ID.NO.16) TTG GTG GGC AGG TAG GTG AGT.

The inventors also combined both methods.

Since 1 and 2 CTCs were detected in 7 (14.8%) and 4 (8.5%) of 47 healthy women, respectively, >1CTC or >2CTCs were selected as cutoffs for further analysis.
Using the above cutoffs, only 7 (18.9%) and 5 (13.5%) of 37 patients with early BC were considered CTC-positive, respectively.

Since no HER2+ CTCs were detected in any of the 47 healthy women, a cutoff of 1 HER2+ CTCs was advantageously chosen.

With this refined cutoff, more women with early BC [11 (29.7%) of 37 women] were considered CTC-positive.

HER2+ CTCs is thus a more sensitive and specific marker for CTCs positivity than total CTCs counts in early breast cancer. Clinical relevance of the above cutoff for CTCs positivity using HER2+ CTCs detection will be performed in ongoing clinical trials of HER2 positive early breast cancer.

### Example 2 Comparison of alternative methods of enrichment and detection of CTC

The inventors measured CTC of 99 patients having early breast cancer or in DCIS state, using the Cellsearch platform. They observed more than 2 CTC in 8 patients. Then they evaluated the Her2 state of the CTC. They observed at least one Her2 positive CTC in 14 patients. However they did not observe any Her 2 positive CTC in the 74 healthy patients tested.

The inventors then developed a method to enrich CTC based on Ficoll gradient, using the same blood samples as for the test with the Cellsearch system.

After the enrichment of CTC in Ficoll, the inventors measured mRNA content of cytokeratin 19 (for instance by PCR using Forward primer: SEQ.ID.NO.17: GCACTACAGCCACTACTACACGA and Reverse primer SEQ.ID.NO.18: CTCATGCGCAGAGCCTGTT) and mammaglobin (for instance in nested PCR using Forward primer1 SEQ.ID.NO.19: CAATCAATCCACAAGTGTCTAA ; Forward primer2 SEQ.ID.NO.20: ACGGATGAAACTCTGAGCAATG; Reverse primer1 SEQ.ID.NO.21: AACATGTATAGCAGGTTTCAAC and Reverse primer2 SEQ.ID.NO.22: CAGTTCTGTGAGCCAAAGGT).

The inventors deduced the presence of CTCs in 12 patients.

Surprisingly, the inventors found only partial correlation between the two methods (some blood samples taken from one patient are positive in one method and negative in the other), evidencing complementarities between the two methods.

The inventors took the advantage to combine the two methods to detect CTC with the measure of Her2 state.

## Claims

1. A cancer diagnostic method which comprises the step of:
- a) submitting a patient blood sample to an enrichment of putative circulating tumor cells;
- b) labelling the retained cells of step a)
for at least one epithelial marker, preferably cytokeratin 8 and/or cytokeratin 18 and/or cytokeratin 19 and/or mammaglobin;
and for HER2 state;
- c) deducing and possibly quantifying a presence of circulating tumor cells in the sample, via a positive signal for epithelial marker detection and
- d) establishing an HER2 state of circulating tumor cells in the said sample.

2. The method of Claim 1, wherein the enrichment of putative circulating tumor cells is obtained upon anti-EPCAM antibodies or specific hyper variable fragments thereof.

3. The method of Claim 1, wherein the enrichment of putative circulating tumor cells is obtained using centrifugation in Ficoll gradients.

4. The method of Claim 1, wherein the enrichment of putative circulating tumor cells is obtained using filtration.

5. The cancer diagnostic method according to any of the previous claims, further comprising a step of labelling the enriched cells of step a
with anti-CD45 antibodies or specific hyper variable fragments thereof and possibly discard the cells labelled with said anti-CD45 antibodies or specific hyper variable fragments thereof.

6. The method according to any of the previous claims, wherein the HER2 state is obtained by detection with anti-HER2 antibodies.

7. The method according to any of the claims 1 to 5, wherein the HER2 state is obtained by quantification of Her2 mRNA content.

8. The method according to any of the claims 1 to 5, wherein the HER2 state is obtained by quantification of gene copies number.

9. The method according to any one of the preceding claims, wherein the sample is obtained from human (woman) at higher risk for developing breast cancer, like a woman with a pre-invasive (cancer) lesion an abnormal mammogram.

10. A cancer diagnostic kit, which comprises an insoluble support, preferably beads, coupled with anti-EpCAM antibodies or specific hyper variable fragments thereof and anti-HER2 antibodies or specific hyper variable fragments thereof and/or means (primers and possibly detection probe) for Her2 mRNA quantification.

11. The kit of claim 10, which further comprise anti-CD45 antibodies or specific hyper variable fragments thereof and antibodies and/or specific hyper variable fragments recognising at least one cytokeratin selected from the group consisting of cytokeratin 8, cytokeratin 18 and cytokeratin 19 and/or mammaglobin.

12. Use of the kit of claim 10 or 11 for a detection of early breast cancer.


SEQUENCE LISTING

<110>  ULB

<120>  Diagnostic method and kit of circulating tumor cells

<130>  ULBB152A

<160>  22

<170>  PatentIn version 3.3

<210>  1
<211>  1724
<212>  DNA
<213>  Homo sapiens


<220>
<221>  CDS
<222>  (35)..(1486)
<223>  Cytokeratin 8

<400>  1
ttcggcaatt cctacctcca ctcctgcctc cacc atg tcc atc agg gtg acc cag      55
aag tcc tac aag gtg tcc acc tct ggc ccc cgg gcc ttc agc agc cgc      103
tcc tac acg agt ggg ccc ggt tcc cgc atc agc tcc tcg agc ttc tcc      151
cga gtg ggc agc agc aac ttt cgc ggt ggc ctg ggc ggc ggc tat ggt      199
ggg gcc agc ggc atg gga ggc atc acc gca gtt acg gtc aac cag agc      247
ctg ctg agc ccc ttg tcc ctg gag gtg gac ccc aac atc cag gcc gtg      295
cgc acc cag gag aag gag cag atc aag acc ctg aac aac aag ttt gcc      343
tcc ttc ata gac aag gta cgg ttc ctg gag cag cag aac aag atg ctg      391
gag acc aag tgg agc ctc ctg cag cag cag aag acg gct cga agc aac      439
atg gac aac atg ttc gag agc tac atc aac aac ctt agg cgg cag ctg      487
gag act ctg ggc cag gag aag ctg aag ctg gag gcg gag ctt ggc aac      535
atg cag ggg ctg gtg gag gac ttc aag aac aag tat gag gat gag atc      583
aat aag cgt aca gag atg gag aac gaa ttt gtc ctc atc aag aag gat      631
gtg gat gaa gca tac atg aac aag gta gag ctg gag tct cgc ctg gaa      679
ggg ctg acc gac gag atc aac ttc ctc agg cag ctg tat gaa gag gag      727
atc cgg gag ctg cag tcc cag atc tcg gac aca tct gtg gtg ctg tcc      775
atg gac aac agc cgc tcc ctg gac atg gag agc atc att gct gag gtc      823
aag gca cag tac gag gat att gcc aac cgc agc cgg gct gag gct gag      871
agc atg tac cag atc aag tat gag gag ctg cag agc ctg gct ggg aag      919
cac ggg gat gac ctg cgg cgc aca aag act gag atc tca gag atg aac      967
cgg aac atc agc cgg ctc cag gct gag att gag ggc ctc aaa ggc cag     1015
agg gct tcc ctg gag gcc gcc att gca gat gcc gag cag cgt gga gag     1063
ctg gcc att aag gat gcc aac gcc aag ttg tcc gag ctg gag gcc gcc     1111
ctg cag cgg gcc aag cag gac atg gcc cgg cag ctg cgt gag tac cag     1159
gag ctg atg aac gtc aag ctg gcc ctg gac atc gac atc gcc acc tac     1207
agg aag ctg ctg gag ggc gag gag agc ccg ctg gag tct ggg atg cag     1255
aac atg agt att cat acg aag acc acc ggc ggc tat gcg ggt ggt ttg     1303
agc tcg gcc tat ggg gac ctc aca gac ccc ggc ctc agc tac agc ctg     1351
ggc tcc agc ttt ggc tct ggc gcg ggc tcc agc tcc ttc agc cgc acc     1399
agc tcc tcc agg gcc gtg gtt gtg aag aag atc gag aca cgt gat ggg     1447
aag ctg gtg tct gag tcc tct gac gtc ctg ccc aag tga acagctgcgg     1496
cagcccctcc cagcctaccc ctcctgcgct gccccagagc ctgggaagga ggccgctatg   1556
cagggtagca ctgggaacag gagacccacc tgaggctcag ccctagccct cagcccacct   1616
ggggagttta ctacctgggg accccccttg cccatgcctc cagctacaaa acaattcaat   1676
tgcttttttt ttttttggtc ccaaaataaa acctcagcta gctctgcc                 1724

<210>  2
<211>  483
<212>  PRT
<213>  Homo sapiens

<400>  2

<210>  3
<211>  1407
<212>  DNA
<213>  Homo sapiens

<220>
<221>  CDS
<222>  (47)..(1339)
<223>  Cytokeratin 18

<400>  3
tcgtccgcaa agcctgagtc ctgtcctttc tctctccccg gacagc atg agc ttc        55
                                                   Met Ser Phe
                                                   1

acc act cgc tcc acc ttc tcc acc aac tac cgg tcc ctg ggc tct gtc      103
Thr Thr Arg Ser Thr Phe Ser Thr Asn Tyr Arg Ser Leu Gly Ser Val
    5                   10                  15

cag gcg ccc agc tac ggc gcc cgg ccg gtc agc agc gcg gcc agc gtc      151
Gln Ala Pro Ser Tyr Gly Ala Arg Pro Val Ser Ser Ala Ala Ser Val
20                  25                  30                  35

tat gca ggc gct ggg ggc tct ggt tcc cgg atc tcc gtg tcc cgc tcc      199
Tyr Ala Gly Ala Gly Gly Ser Gly Ser Arg Ile Ser Val Ser Arg Ser
                40                  45                  50

acc agc ttc agg ggc ggc atg ggg tcc ggg ggc ctg gcc acc ggg ata      247
Thr Ser Phe Arg Gly Gly Met Gly Ser Gly Gly Leu Ala Thr Gly Ile
            55                  60                  65

gcc ggg ggt ctg gca gga atg gga ggc atc cag aac gag aag gag acc      295
Ala Gly Gly Leu Ala Gly Met Gly Gly Ile Gln Asn Glu Lys Glu Thr
        70                  75                  80

atg caa agc ctg aac gac cgc ctg gcc tct tac ctg gac aga gtg agg      343
Met Gln Ser Leu Asn Asp Arg Leu Ala Ser Tyr Leu Asp Arg Val Arg
    85                  90                  95

agc ctg gag acc gag aac cgg agg ctg gag agc aaa atc cgg gag cac      391
Ser Leu Glu Thr Glu Asn Arg Arg Leu Glu Ser Lys Ile Arg Glu His
100                 105                 110                 115

ttg gag aag aag gga ccc cag gtc aga gac tgg agc cat tac ttc aag      439
Leu Glu Lys Lys Gly Pro Gln Val Arg Asp Trp Ser His Tyr Phe Lys
                120                 125                 130

atc atc gag gac ctg agg gct cag atc ttc gca aat act gtg gac aat      487
Ile Ile Glu Asp Leu Arg Ala Gln Ile Phe Ala Asn Thr Val Asp Asn
            135                 140                 145

gcc cgc atc gtt ctg cag att gac aat gcc cgt ctt gct gct gat gac      535
Ala Arg Ile Val Leu Gln Ile Asp Asn Ala Arg Leu Ala Ala Asp Asp
        150                 155                 160

ttt aga gtc aag tat gag aca gag ctg gcc atg cgc cag tct gtg gag      583

Phe Arg Val Lys Tyr Glu Thr Glu Leu Ala Met Arg Gln Ser Val Glu
    165                 170                 175

aac gac atc cat ggg ctc cgc aag gtc att gat gac acc aat atc aca         631
Asn Asp Ile His Gly Leu Arg Lys Val Ile Asp Asp Thr Asn Ile Thr
180                 185                 190                 195

cga ctg cag ctg gag aca gag atc gag gct ctc aag gag gag ctg ctc         679
Arg Leu Gln Leu Glu Thr Glu Ile Glu Ala Leu Lys Glu Glu Leu Leu
                200                 205                 210

ttc atg aag aag aac cac gaa gag gaa gta aaa ggc cta caa gcc cag         727
Phe Met Lys Lys Asn His Glu Glu Glu Val Lys Gly Leu Gln Ala Gln
            215                 220                 225

att gcc agc tct ggg ttg acc gtg gag gta gat gcc ccc aaa tct cag         775
Ile Ala Ser Ser Gly Leu Thr Val Glu Val Asp Ala Pro Lys Ser Gln
        230                 235                 240

gac ctc gcc aag atc atg gca gac atc cgg gcc caa tat gac gag ctg         823
Asp Leu Ala Lys Ile Met Ala Asp Ile Arg Ala Gln Tyr Asp Glu Leu
    245                 250                 255

gct cgg aag aac cga gag gag cta gac aag tac tgg tct cag cag att         871
Ala Arg Lys Asn Arg Glu Glu Leu Asp Lys Tyr Trp Ser Gln Gln Ile
260                 265                 270                 275

gag gag agc acc aca gtg gtc acc aca cag tct gct gag gtt gga gct         919
Glu Glu Ser Thr Thr Val Val Thr Thr Gln Ser Ala Glu Val Gly Ala
                280                 285                 290

gct gag acg acg ctc aca gag ctg aga cgt aca gtc cag tcc ttg gag         967
Ala Glu Thr Thr Leu Thr Glu Leu Arg Arg Thr Val Gln Ser Leu Glu
            295                 300                 305

atc gac ctg gac tcc atg aga aat ctg aag gcc agc ttg gag aac agc        1015
Ile Asp Leu Asp Ser Met Arg Asn Leu Lys Ala Ser Leu Glu Asn Ser
        310                 315                 320

ctg agg gag gtg gag gcc cgc tac gcc cta cag atg gag cag ctc aac        1063
Leu Arg Glu Val Glu Ala Arg Tyr Ala Leu Gln Met Glu Gln Leu Asn
    325                 330                 335

ggg atc ctg ctg cac ctt gag tca gag ctg gca cag acc cgg gca gag        1111
Gly Ile Leu Leu His Leu Glu Ser Glu Leu Ala Gln Thr Arg Ala Glu
340                 345                 350                 355

gga cag cgc cag gcc cag gag tat gag gcc ctg ctg aac atc aag gtc        1159
Gly Gln Arg Gln Ala Gln Glu Tyr Glu Ala Leu Leu Asn Ile Lys Val
                360                 365                 370

aag ctg gag gct gag atc gcc acc tac cgc cgc ctg ctg gaa gat ggc        1207
Lys Leu Glu Ala Glu Ile Ala Thr Tyr Arg Arg Leu Leu Glu Asp Gly
            375                 380                 385

gag gac ttt aat ctt ggt gat gcc ttg gac agc agc aac tcc atg caa        1255
Glu Asp Phe Asn Leu Gly Asp Ala Leu Asp Ser Ser Asn Ser Met Gln
        390                 395                 400

acc atc caa aag acc acc acc cgc cgg ata gtg gat ggc aaa gtg gtg        1303
Thr Ile Gln Lys Thr Thr Thr Arg Arg Ile Val Asp Gly Lys Val Val
    405                 410                 415

tct gag acc aat gac acc aaa gtt ctg agg cat taa gccagcagaa             1349
Ser Glu Thr Asn Asp Thr Lys Val Leu Arg His
420                 425                 430

gcagggtacc ctttggggag caggaggcca ataaaaagtt cagagttcat tggatgtc        1407

<210>  4
<211>  430
<212>  PRT
<213>  Homo sapiens

<400>  4

Met Ser Phe Thr Thr Arg Ser Thr Phe Ser Thr Asn Tyr Arg Ser Leu
1               5                   10                  15

Gly Ser Val Gln Ala Pro Ser Tyr Gly Ala Arg Pro Val Ser Ser Ala
            20                  25                  30

Ala Ser Val Tyr Ala Gly Ala Gly Gly Ser Gly Ser Arg Ile Ser Val
        35                  40                  45

Ser Arg Ser Thr Ser Phe Arg Gly Gly Met Gly Ser Gly Gly Leu Ala
    50                  55                  60

Thr Gly Ile Ala Gly Gly Leu Ala Gly Met Gly Gly Ile Gln Asn Glu
65                  70                  75                  80

Lys Glu Thr Met Gln Ser Leu Asn Asp Arg Leu Ala Ser Tyr Leu Asp
                85                  90                  95

Arg Val Arg Ser Leu Glu Thr Glu Asn Arg Arg Leu Glu Ser Lys Ile
            100                 105                 110

Arg Glu His Leu Glu Lys Lys Gly Pro Gln Val Arg Asp Trp Ser His
        115                 120                 125

Tyr Phe Lys Ile Ile Glu Asp Leu Arg Ala Gln Ile Phe Ala Asn Thr
    130                 135                 140

Val Asp Asn Ala Arg Ile Val Leu Gln Ile Asp Asn Ala Arg Leu Ala
145                 150                 155                 160

Ala Asp Asp Phe Arg Val Lys Tyr Glu Thr Glu Leu Ala Met Arg Gln
                165                 170                 175

Ser Val Glu Asn Asp Ile His Gly Leu Arg Lys Val Ile Asp Asp Thr
            180                 185                 190

Asn Ile Thr Arg Leu Gln Leu Glu Thr Glu Ile Glu Ala Leu Lys Glu
        195                 200                 205

Glu Leu Leu Phe Met Lys Lys Asn His Glu Glu Glu Val Lys Gly Leu
    210                 215                 220

Gln Ala Gln Ile Ala Ser Ser Gly Leu Thr Val Glu Val Asp Ala Pro
225                 230                 235                 240

Lys Ser Gln Asp Leu Ala Lys Ile Met Ala Asp Ile Arg Ala Gln Tyr

245                 250                 255

Asp Glu Leu Ala Arg Lys Asn Arg Glu Glu Leu Asp Lys Tyr Trp Ser
            260                 265                 270

Gln Gln Ile Glu Glu Ser Thr Thr Val Val Thr Thr Gln Ser Ala Glu
        275                 280                 285

Val Gly Ala Ala Glu Thr Thr Leu Thr Glu Leu Arg Arg Thr Val Gln
    290                 295                 300

Ser Leu Glu Ile Asp Leu Asp Ser Met Arg Asn Leu Lys Ala Ser Leu
305                 310                 315                 320

Glu Asn Ser Leu Arg Glu Val Glu Ala Arg Tyr Ala Leu Gln Met Glu
                325                 330                 335

Gln Leu Asn Gly Ile Leu Leu His Leu Glu Ser Glu Leu Ala Gln Thr
            340                 345                 350

Arg Ala Glu Gly Gln Arg Gln Ala Gln Glu Tyr Glu Ala Leu Leu Asn
        355                 360                 365

Ile Lys Val Lys Leu Glu Ala Glu Ile Ala Thr Tyr Arg Arg Leu Leu
    370                 375                 380

Glu Asp Gly Glu Asp Phe Asn Leu Gly Asp Ala Leu Asp Ser Ser Asn
385                 390                 395                 400

Ser Met Gln Thr Ile Gln Lys Thr Thr Thr Arg Arg Ile Val Asp Gly
                405                 410                 415

Lys Val Val Ser Glu Thr Asn Asp Thr Lys Val Leu Arg His
            420                 425                 430

<210>  5
<211>  1490
<212>  DNA
<213>  Homo sapiens

<220>
<221>  CDS
<222>  (143)..(1345)
<223>  Cytokeratin 19

<400>  5
agatatccgc ccctgacacc attcctccct tcccccctcc accggccgcg ggcataaaag      60

gcgccaggtg agggcctcgc cgctcctccc gcgaatcgca gcttctgaga ccagggttgc     120

tccgtccgtg ctccgcctcg cc atg act tcc tac agc tat cgc cag tcg tcg      172
                         Met Thr Ser Tyr Ser Tyr Arg Gln Ser Ser
                         1               5                   10

gcc acg tcg tcc ttc gga ggc ctg ggc ggc ggc tcc gtg cgt ttt ggg      220
Ala Thr Ser Ser Phe Gly Gly Leu Gly Gly Gly Ser Val Arg Phe Gly

                  15                  20                  25

ccg ggg gtc gcc ttt cgc gcg ccc agc att cac ggg ggc tcc ggc ggc      268
Pro Gly Val Ala Phe Arg Ala Pro Ser Ile His Gly Gly Ser Gly Gly
            30                  35                  40

cgc ggc gta tcc gtg tcc tcc gcc cgc ttt gtg tcc tcg tcc tcc tcg      316
Arg Gly Val Ser Val Ser Ser Ala Arg Phe Val Ser Ser Ser Ser Ser
        45                  50                  55

ggg gcc tac ggc ggc ggc tac ggc ggc gtc ctg acc gcg tcc gac ggg      364
Gly Ala Tyr Gly Gly Gly Tyr Gly Gly Val Leu Thr Ala Ser Asp Gly
    60                  65                  70

ctg ctg gcg ggc aac gag aag cta acc atg cag aac ctc aac gac cgc      412
Leu Leu Ala Gly Asn Glu Lys Leu Thr Met Gln Asn Leu Asn Asp Arg
75                  80                  85                  90

ctg gcc tcc tac ctg gac aag gtg cgc gcc ctg gag gcg gcc aac ggc      460
Leu Ala Ser Tyr Leu Asp Lys Val Arg Ala Leu Glu Ala Ala Asn Gly
                95                  100                 105

gag cta gag gtg aag atc cgc gac tgg tac cag aag cag ggg cct ggg      508
Glu Leu Glu Val Lys Ile Arg Asp Trp Tyr Gln Lys Gln Gly Pro Gly
            110                 115                 120

ccc tcc cgc gac tac agc cac tac tac acg acc atc cag gac ctg cgg      556
Pro Ser Arg Asp Tyr Ser His Tyr Tyr Thr Thr Ile Gln Asp Leu Arg
        125                 130                 135

gac aag att ctt ggt gcc acc att gag aac tcc agg att gtc ctg cag      604
Asp Lys Ile Leu Gly Ala Thr Ile Glu Asn Ser Arg Ile Val Leu Gln
    140                 145                 150

atc gac aat gcc cgt ctg gct gca gat gac ttc cga acc aag ttt gag      652
Ile Asp Asn Ala Arg Leu Ala Ala Asp Asp Phe Arg Thr Lys Phe Glu
155                 160                 165                 170

acg gaa cag gct ctg cgc atg agc gtg gag gcc gac atc aac ggc ctg      700
Thr Glu Gln Ala Leu Arg Met Ser Val Glu Ala Asp Ile Asn Gly Leu
                175                 180                 185

cgc agg gtg ctg gat gag ctg acc ctg gcc agg acc gac ctg gag atg      748
Arg Arg Val Leu Asp Glu Leu Thr Leu Ala Arg Thr Asp Leu Glu Met
            190                 195                 200

cag atc gaa ggc ctg aag gaa gag ctg gcc tac ctg aag aag aac cat      796
Gln Ile Glu Gly Leu Lys Glu Glu Leu Ala Tyr Leu Lys Lys Asn His
        205                 210                 215

gag gag gaa atc agt acg ctg agg ggc caa gtg gga ggc cag gtc agt      844
Glu Glu Glu Ile Ser Thr Leu Arg Gly Gln Val Gly Gly Gln Val Ser
    220                 225                 230

gtg gag gtg gat tcc gct ccg ggc acc gat ctc gcc aag atc ctg agt      892
Val Glu Val Asp Ser Ala Pro Gly Thr Asp Leu Ala Lys Ile Leu Ser
235                 240                 245                 250

gac atg cga agc caa tat gag gtc atg gcc gag cag aac cgg aag gat      940
Asp Met Arg Ser Gln Tyr Glu Val Met Ala Glu Gln Asn Arg Lys Asp
                255                 260                 265

gct gaa gcc tgg ttc acc agc cgg act gaa gaa ttg aac cgg gag gtc      988
Ala Glu Ala Trp Phe Thr Ser Arg Thr Glu Glu Leu Asn Arg Glu Val
            270                 275                 280

gct ggc cac acg gag cag ctc cag atg agc agg tcc gag gtt act gac     1036
Ala Gly His Thr Glu Gln Leu Gln Met Ser Arg Ser Glu Val Thr Asp
        285                 290                 295

ctg cgg cgc acc ctt cag ggt ctt gag att gag ctg cag tca cag ctg     1084
Leu Arg Arg Thr Leu Gln Gly Leu Glu Ile Glu Leu Gln Ser Gln Leu
    300                 305                 310

agc atg aaa gct gcc ttg gaa gac aca ctg gca gaa acg gag gcg cgc     1132
Ser Met Lys Ala Ala Leu Glu Asp Thr Leu Ala Glu Thr Glu Ala Arg
315                 320                 325                 330

ttt gga gcc cag ctg gcg cat atc cag gcg ctg atc agc ggt att gaa     1180
Phe Gly Ala Gln Leu Ala His Ile Gln Ala Leu Ile Ser Gly Ile Glu
                335                 340                 345

gcc cag ctg ggc gat gtg cga gct gat agt gag cgg cag aat cag gag     1228
Ala Gln Leu Gly Asp Val Arg Ala Asp Ser Glu Arg Gln Asn Gln Glu
            350                 355                 360

tac cag cgg ctc atg gac atc aag tcg cgg ctg gag cag gag att gcc     1276
Tyr Gln Arg Leu Met Asp Ile Lys Ser Arg Leu Glu Gln Glu Ile Ala
        365                 370                 375

acc tac cgc agc ctg ctc gag gga cag gaa gat cac tac aac aat ttg     1324
Thr Tyr Arg Ser Leu Leu Glu Gly Gln Glu Asp His Tyr Asn Asn Leu
    380                 385                 390

tct gcc tcc aag gtc ctc tga ggcagcaggc tctggggctt ctgctgtcct     1375
Ser Ala Ser Lys Val Leu
395                 400

ttggagggtg tcttctgggt agagggatgg gaaggaaggg acccttaccc ccggctcttc     1435

tcctgacctg ccaataaaaa tttatggtcc aagggaaaaa aaaaaaaaaa aaaaa     1490


<210>  6
<211>  400
<212>  PRT
<213>  Homo sapiens

<400>  6

Met Thr Ser Tyr Ser Tyr Arg Gln Ser Ser Ala Thr Ser Ser Phe Gly
1               5                   10                  15


Gly Leu Gly Gly Gly Ser Val Arg Phe Gly Pro Gly Val Ala Phe Arg
            20                  25                  30


Ala Pro Ser Ile His Gly Gly Ser Gly Gly Arg Gly Val Ser Val Ser
        35                  40                  45


Ser Ala Arg Phe Val Ser Ser Ser Ser Ser Gly Ala Tyr Gly Gly Gly
    50                  55                  60


Tyr Gly Gly Val Leu Thr Ala Ser Asp Gly Leu Leu Ala Gly Asn Glu
65                  70                  75                  80


Lys Leu Thr Met Gln Asn Leu Asn Asp Arg Leu Ala Ser Tyr Leu Asp
                85                  90                  95


Lys Val Arg Ala Leu Glu Ala Ala Asn Gly Glu Leu Glu Val Lys Ile
            100                 105                 110

Arg Asp Trp Tyr Gln Lys Gln Gly Pro Gly Pro Ser Arg Asp Tyr Ser
        115                 120                 125

His Tyr Tyr Thr Thr Ile Gln Asp Leu Arg Asp Lys Ile Leu Gly Ala
    130                 135                 140

Thr Ile Glu Asn Ser Arg Ile Val Leu Gln Ile Asp Asn Ala Arg Leu
145                 150                 155                 160

Ala Ala Asp Asp Phe Arg Thr Lys Phe Glu Thr Glu Gln Ala Leu Arg
                165                 170                 175

Met Ser Val Glu Ala Asp Ile Asn Gly Leu Arg Arg Val Leu Asp Glu
            180                 185                 190

Leu Thr Leu Ala Arg Thr Asp Leu Glu Met Gln Ile Glu Gly Leu Lys
        195                 200                 205

Glu Glu Leu Ala Tyr Leu Lys Lys Asn His Glu Glu Glu Ile Ser Thr
    210                 215                 220

Leu Arg Gly Gln Val Gly Gly Gln Val Ser Val Glu Val Asp Ser Ala
225                 230                 235                 240

Pro Gly Thr Asp Leu Ala Lys Ile Leu Ser Asp Met Arg Ser Gln Tyr
                245                 250                 255

Glu Val Met Ala Glu Gln Asn Arg Lys Asp Ala Glu Ala Trp Phe Thr
            260                 265                 270

Ser Arg Thr Glu Glu Leu Asn Arg Glu Val Ala Gly His Thr Glu Gln
        275                 280                 285

Leu Gln Met Ser Arg Ser Glu Val Thr Asp Leu Arg Arg Thr Leu Gln
    290                 295                 300

Gly Leu Glu Ile Glu Leu Gln Ser Gln Leu Ser Met Lys Ala Ala Leu
305                 310                 315                 320

Glu Asp Thr Leu Ala Glu Thr Glu Ala Arg Phe Gly Ala Gln Leu Ala
                325                 330                 335

His Ile Gln Ala Leu Ile Ser Gly Ile Glu Ala Gln Leu Gly Asp Val
            340                 345                 350

Arg Ala Asp Ser Glu Arg Gln Asn Gln Glu Tyr Gln Arg Leu Met Asp
        355                 360                 365

Ile Lys Ser Arg Leu Glu Gln Glu Ile Ala Thr Tyr Arg Ser Leu Leu
    370                 375                 380

Glu Gly Gln Glu Asp His Tyr Asn Asn Leu Ser Ala Ser Lys Val Leu

385                 390                 395                 400

<210>  7
<211>  4597
<212>  DNA
<213>  Homo sapiens

<220>
<221>  CDS
<222>  (147)..(3578)
<223>  CD 45

<400>  7
cgacatttta actgaactgc gggataaagt gaaatctttc cgtgcagctc tacgagagga      60

ggaaattgtt cctcgtctga taagacaaca gtggagaaag gacgcatgct gtttcttagg     120

gacacggctg acttccagat atgacc atg tat ttg tgg ctt aaa ctc ttg gca      173
                             Met Tyr Leu Trp Leu Lys Leu Leu Ala
                             1               5

ttt ggc ttt gcc ttt ctg gac aca gaa gta ttt gtg aca ggg caa agc      221
Phe Gly Phe Ala Phe Leu Asp Thr Glu Val Phe Val Thr Gly Gln Ser
10                  15                  20                  25

cca aca cct tcc ccc act gat gcc tac ctt aat gcc tct gaa aca acc      269
Pro Thr Pro Ser Pro Thr Asp Ala Tyr Leu Asn Ala Ser Glu Thr Thr
                30                  35                  40

act ctg agc cct tct gga agc gct gtc att tca acc aca aca ata gct      317
Thr Leu Ser Pro Ser Gly Ser Ala Val Ile Ser Thr Thr Thr Ile Ala
            45                  50                  55

act act cca tct aag cca aca tgt gat gaa aaa tat gca aac atc act      365
Thr Thr Pro Ser Lys Pro Thr Cys Asp Glu Lys Tyr Ala Asn Ile Thr
        60                  65                  70

gtg gat tac tta tat aac aag gaa act aaa tta ttt aca gca aag cta      413
Val Asp Tyr Leu Tyr Asn Lys Glu Thr Lys Leu Phe Thr Ala Lys Leu
    75                  80                  85

aat gtt aat gag aat gtg gaa tgt gga aac aat act tgc aca aac aat      461
Asn Val Asn Glu Asn Val Glu Cys Gly Asn Asn Thr Cys Thr Asn Asn
90                  95                  100                 105

gag gtg cat aac ctt aca gaa tgt aaa aat gcg tct gtt tcc ata tct      509
Glu Val His Asn Leu Thr Glu Cys Lys Asn Ala Ser Val Ser Ile Ser
                110                 115                 120

cat aat tca tgt act gct cct gat aag aca tta ata tta gat gtg cca      557
His Asn Ser Cys Thr Ala Pro Asp Lys Thr Leu Ile Leu Asp Val Pro
            125                 130                 135

cca ggg gtt gaa aag ttt cag tta cat gat tgt aca caa gtt gaa aaa      605
Pro Gly Val Glu Lys Phe Gln Leu His Asp Cys Thr Gln Val Glu Lys
        140                 145                 150

gca gat act act att tgt tta aaa tgg aaa aat att gaa acc ttt act      653
Ala Asp Thr Thr Ile Cys Leu Lys Trp Lys Asn Ile Glu Thr Phe Thr
    155                 160                 165

tgt gat aca cag aat att acc tac aga ttt cag tgt ggt aat atg ata      701
Cys Asp Thr Gln Asn Ile Thr Tyr Arg Phe Gln Cys Gly Asn Met Ile
170                 175                 180                 185

ttt gat aat aaa gaa att aaa tta gaa aac ctt gaa ccc gaa cat gag      749
Phe Asp Asn Lys Glu Ile Lys Leu Glu Asn Leu Glu Pro Glu His Glu

                    190                 195                 200

tat aag tgt gac tca gaa ata ctc tat aat aac cac aag ttt act aac        797
Tyr Lys Cys Asp Ser Glu Ile Leu Tyr Asn Asn His Lys Phe Thr Asn
            205                 210                 215

gca agt aaa att att aaa aca gat ttt ggg agt cca gga gag cct cag        845
Ala Ser Lys Ile Ile Lys Thr Asp Phe Gly Ser Pro Gly Glu Pro Gln
        220                 225                 230

att att ttt tgt aga agt gaa gct gca cat caa gga gta att acc tgg        893
Ile Ile Phe Cys Arg Ser Glu Ala Ala His Gln Gly Val Ile Thr Trp
    235                 240                 245

aat ccc cct caa aga tca ttt cat aat ttt acc ctc tgt tat ata aaa        941
Asn Pro Pro Gln Arg Ser Phe His Asn Phe Thr Leu Cys Tyr Ile Lys
250                 255                 260                 265

gag aca gaa aaa gat tgc ctc aat ctg gat aaa aac ctg atc aaa tat        989
Glu Thr Glu Lys Asp Cys Leu Asn Leu Asp Lys Asn Leu Ile Lys Tyr
                270                 275                 280

gat ttg caa aat tta aaa cct tat acg aaa tat gtt tta tca tta cat       1037
Asp Leu Gln Asn Leu Lys Pro Tyr Thr Lys Tyr Val Leu Ser Leu His
            285                 290                 295

gcc tac atc att gca aaa gtg caa cgt aat gga agt gct gca atg tgt       1085
Ala Tyr Ile Ile Ala Lys Val Gln Arg Asn Gly Ser Ala Ala Met Cys
        300                 305                 310

cat ttc aca act aaa agt gct cct cca agc cag gtc tgg aac atg act       1133
His Phe Thr Thr Lys Ser Ala Pro Pro Ser Gln Val Trp Asn Met Thr
    315                 320                 325

gtc tcc atg aca tca gat aat agt atg cat gtc aag tgt agg cct ccc       1181
Val Ser Met Thr Ser Asp Asn Ser Met His Val Lys Cys Arg Pro Pro
330                 335                 340                 345

agg gac cgt aat ggc ccc cat gaa cgt tac cat ttg gaa gtt gaa gct       1229
Arg Asp Arg Asn Gly Pro His Glu Arg Tyr His Leu Glu Val Glu Ala
                350                 355                 360

gga aat act ctg gtt aga aat gag tcg cat aag aat tgc gat ttc cgt       1277
Gly Asn Thr Leu Val Arg Asn Glu Ser His Lys Asn Cys Asp Phe Arg
            365                 370                 375

gta aaa gat ctt caa tat tca aca gac tac act ttt aag gcc tat ttt       1325
Val Lys Asp Leu Gln Tyr Ser Thr Asp Tyr Thr Phe Lys Ala Tyr Phe
        380                 385                 390

cac aat gga gac tat cct gga gaa ccc ttt att tta cat cat tca aca       1373
His Asn Gly Asp Tyr Pro Gly Glu Pro Phe Ile Leu His His Ser Thr
    395                 400                 405

tct tat aat tct aag gca ctg ata gca ttt ctg gca ttt ctg att att       1421
Ser Tyr Asn Ser Lys Ala Leu Ile Ala Phe Leu Ala Phe Leu Ile Ile
410                 415                 420                 425

gtg aca tca ata gcc ctg ctt gtt gtt ctc tac aaa atc tat gat cta       1469
Val Thr Ser Ile Ala Leu Leu Val Val Leu Tyr Lys Ile Tyr Asp Leu
                430                 435                 440

cat aag aaa aga tcc tgc aat tta gat gaa cag cag gag ctt gtt gaa       1517
His Lys Lys Arg Ser Cys Asn Leu Asp Glu Gln Gln Glu Leu Val Glu
            445                 450                 455

agg gat gat gaa aaa caa ctg atg aat gtg gag cca atc cat gca gat       1565
Arg Asp Asp Glu Lys Gln Leu Met Asn Val Glu Pro Ile His Ala Asp
        460                 465                 470

att ttg ttg gaa act tat aag agg aag att gct gat gaa gga aga ctt 1613
Ile Leu Leu Glu Thr Tyr Lys Arg Lys Ile Ala Asp Glu Gly Arg Leu
    475                 480                 485

ttt ctg gct gaa ttt cag agc atc ccg cgg gtg ttc agc aag ttt cct 1661
Phe Leu Ala Glu Phe Gln Ser Ile Pro Arg Val Phe Ser Lys Phe Pro
490                 495                 500                 505

ata aag gaa gct cga aag ccc ttt aac cag aat aaa aac cgt tat gtt 1709
Ile Lys Glu Ala Arg Lys Pro Phe Asn Gln Asn Lys Asn Arg Tyr Val
                510                 515                 520

gac att ctt cct tat gat tat aac cgt gtt gaa ctc tct gag ata aac 1757
Asp Ile Leu Pro Tyr Asp Tyr Asn Arg Val Glu Leu Ser Glu Ile Asn
            525                 530                 535

gga gat gca ggg tca aac tac ata aat gcc agc tat att gat ggt ttc 1805
Gly Asp Ala Gly Ser Asn Tyr Ile Asn Ala Ser Tyr Ile Asp Gly Phe
        540                 545                 550

aaa gaa ccc agg aaa tac att gct gca caa ggt ccc agg gat gaa act 1853
Lys Glu Pro Arg Lys Tyr Ile Ala Ala Gln Gly Pro Arg Asp Glu Thr
    555                 560                 565

gtt gat gat ttc tgg agg atg att tgg gaa cag aaa gcc aca gtt att 1901
Val Asp Asp Phe Trp Arg Met Ile Trp Glu Gln Lys Ala Thr Val Ile
570                 575                 580                 585

gtc atg gtc act cga tgt gaa gaa gga aac agg aac aag tgt gca gaa 1949
Val Met Val Thr Arg Cys Glu Glu Gly Asn Arg Asn Lys Cys Ala Glu
                590                 595                 600

tac tgg ccg tca atg gaa gag ggc act cgg gct ttt gga gat gtt gtt 1997
Tyr Trp Pro Ser Met Glu Glu Gly Thr Arg Ala Phe Gly Asp Val Val
            605                 610                 615

gta aag atc aac cag cac aaa aga tgt cca gat tac atc att cag aaa 2045
Val Lys Ile Asn Gln His Lys Arg Cys Pro Asp Tyr Ile Ile Gln Lys
        620                 625                 630

ttg aac att gta aat aaa aaa gaa aaa gca act gga aga gag gtg act 2093
Leu Asn Ile Val Asn Lys Lys Glu Lys Ala Thr Gly Arg Glu Val Thr
    635                 640                 645

cac att cag ttc acc agc tgg cca gac cac ggg gtg cct gag gat cct 2141
His Ile Gln Phe Thr Ser Trp Pro Asp His Gly Val Pro Glu Asp Pro
650                 655                 660                 665

cac ttg ctc ctc aaa ctg aga agg aga gtg aat gcc ttc agc aat ttc 2189
His Leu Leu Leu Lys Leu Arg Arg Arg Val Asn Ala Phe Ser Asn Phe
                670                 675                 680

ttc agt ggt ccc att gtg gtg cac tgc agt gct ggt gtt ggg cgc aca 2237
Phe Ser Gly Pro Ile Val Val His Cys Ser Ala Gly Val Gly Arg Thr
            685                 690                 695

gga acc tat atc gga att gat gcc atg cta gaa ggc ctg gaa gcc gag 2285
Gly Thr Tyr Ile Gly Ile Asp Ala Met Leu Glu Gly Leu Glu Ala Glu
        700                 705                 710

aac aaa gtg gat gtt tat ggt tat gtt gtc aag cta agg cga cag aga 2333
Asn Lys Val Asp Val Tyr Gly Tyr Val Val Lys Leu Arg Arg Gln Arg
    715                 720                 725

tgc ctg atg gtt caa gta gag gcc cag tac atc ttg atc cat cag gct 2381
Cys Leu Met Val Gln Val Glu Ala Gln Tyr Ile Leu Ile His Gln Ala
730                 735                 740                 745

ttg gtg gaa tac aat cag ttt gga gaa aca gaa gtg aat ttg tct gaa     2429
Leu Val Glu Tyr Asn Gln Phe Gly Glu Thr Glu Val Asn Leu Ser Glu
                750                 755                 760

tta cat cca tat cta cat aac atg aag aaa agg gat cca ccc agt gag     2477
Leu His Pro Tyr Leu His Asn Met Lys Lys Arg Asp Pro Pro Ser Glu
            765                 770                 775

ccg tct cca cta gag gct gaa ttc cag aga ctt cct tca tat agg agc     2525
Pro Ser Pro Leu Glu Ala Glu Phe Gln Arg Leu Pro Ser Tyr Arg Ser
        780                 785                 790

tgg agg aca cag cac att gga aat caa gaa gaa aat aaa agt aaa aac     2573
Trp Arg Thr Gln His Ile Gly Asn Gln Glu Glu Asn Lys Ser Lys Asn
    795                 800                 805

agg aat tct aat gtc atc cca tat gac tat aac aga gtg cca ctt aaa     2621
Arg Asn Ser Asn Val Ile Pro Tyr Asp Tyr Asn Arg Val Pro Leu Lys
810                 815                 820                 825

cat gag ctg gaa atg agt aaa gag agt gag cat gat tca gat gaa tcc     2669
His Glu Leu Glu Met Ser Lys Glu Ser Glu His Asp Ser Asp Glu Ser
                830                 835                 840

tct gat gat gac agt gat tca gag gaa cca agc aaa tac atc aat gca     2717
Ser Asp Asp Asp Ser Asp Ser Glu Glu Pro Ser Lys Tyr Ile Asn Ala
            845                 850                 855

tct ttt ata atg agc tac tgg aaa cct gaa gtg atg att gct gct cag     2765
Ser Phe Ile Met Ser Tyr Trp Lys Pro Glu Val Met Ile Ala Ala Gln
        860                 865                 870

gga cca ctg aag gag acc att ggt gac ttt tgg cag atg atc ttc caa     2813
Gly Pro Leu Lys Glu Thr Ile Gly Asp Phe Trp Gln Met Ile Phe Gln
    875                 880                 885

aga aaa gtc aaa gtt att gtt atg ctg aca gaa ctg aaa cat gga gac     2861
Arg Lys Val Lys Val Ile Val Met Leu Thr Glu Leu Lys His Gly Asp
890                 895                 900                 905

cag gaa atc tgt gct cag tac tgg gga gaa gga aag caa aca tat gga     2909
Gln Glu Ile Cys Ala Gln Tyr Trp Gly Glu Gly Lys Gln Thr Tyr Gly
                910                 915                 920

gat att gaa gtt gac ctg aaa gac aca gac aaa tct tca act tat acc     2957
Asp Ile Glu Val Asp Leu Lys Asp Thr Asp Lys Ser Ser Thr Tyr Thr
            925                 930                 935

ctt cgt gtc ttt gaa ctg aga cat tcc aag agg aaa gac tct cga act     3005
Leu Arg Val Phe Glu Leu Arg His Ser Lys Arg Lys Asp Ser Arg Thr
        940                 945                 950

gtg tac cag tac caa tat aca aac tgg agt gtg gag cag ctt cct gca     3053
Val Tyr Gln Tyr Gln Tyr Thr Asn Trp Ser Val Glu Gln Leu Pro Ala
    955                 960                 965

gaa ccc aag gaa tta atc tct atg att cag gtc gtc aaa caa aaa ctt     3101
Glu Pro Lys Glu Leu Ile Ser Met Ile Gln Val Val Lys Gln Lys Leu
970                 975                 980                 985

ccc cag aag aat tcc tct gaa ggg aac aag cat cac aag agt aca  cct     3149
Pro Gln Lys Asn Ser Ser Glu Gly Asn Lys His His Lys Ser Thr  Pro
                990                 995                 1000

cta ctc att cac  tgc agg gat gga tct  cag caa acg gga ata  ttt     3194
Leu Leu Ile His  Cys Arg Asp Gly Ser  Gln Gln Thr Gly Ile  Phe
            1005                1010                1015

tgt gct ttg tta  aat ctc tta gaa agt  gcg gaa aca gaa gag  gta     3239

Cys Ala Leu Leu Asn Leu Leu Glu Ser Ala Glu Thr Glu Glu Val
            1020                1025                1030

gtg gat att ttt caa gtg gta aaa gct cta cgc aaa gct agg cca      3284
Val Asp Ile Phe Gln Val Val Lys Ala Leu Arg Lys Ala Arg Pro
            1035                1040                1045

ggc atg gtt tcc aca ttc gag caa tat caa ttc cta tat gac gtc      3329
Gly Met Val Ser Thr Phe Glu Gln Tyr Gln Phe Leu Tyr Asp Val
            1050                1055                1060

att gcc agc acc tac cct gct cag aat gga caa gta aag aaa aac      3374
Ile Ala Ser Thr Tyr Pro Ala Gln Asn Gly Gln Val Lys Lys Asn
            1065                1070                1075

aac cat caa gaa gat aaa att gaa ttt gat aat gaa gtg gac aaa      3419
Asn His Gln Glu Asp Lys Ile Glu Phe Asp Asn Glu Val Asp Lys
            1080                1085                1090

gta aag cag gat gct aat tgt gtt aat cca ctt ggt gcc cca gaa      3464
Val Lys Gln Asp Ala Asn Cys Val Asn Pro Leu Gly Ala Pro Glu
            1095                1100                1105

aag ctc cct gaa gca aag gaa cag gct gaa ggt tct gaa ccc acg      3509
Lys Leu Pro Glu Ala Lys Glu Gln Ala Glu Gly Ser Glu Pro Thr
            1110                1115                1120

agt ggc act gag ggg cca gaa cat tct gtc aat ggt cct gca agt      3554
Ser Gly Thr Glu Gly Pro Glu His Ser Val Asn Gly Pro Ala Ser
            1125                1130                1135

cca gcc tta aat caa ggt tca tag gaaaagacat aaatgaggaa actccaaacc      3608
Pro Ala Leu Asn Gln Gly Ser
            1140

tcctgttagc tgttatttct atttttgtag aagtaggaag tgaaaatagg tatacagtgg      3668

attaattaaa tgcagcgaac caatatttgt agaagggtta tattttacta ctgtggaaaa      3728

atatttaaga tagttttgcc agaacagttt gtacagacgt atgcttattt taaaatttta      3788

tctcttattc agtaaaaaac aacttctttg taatcgttat gtgtgtatat gtatgtgtgt      3848

atgggtgtgt gtttgtgtga gagacagaga aagagagaga attctttcaa gtgaatctaa      3908

aagcttttgc ttttcctttg tttttatgaa gaaaaaatac attttatatt agaagtgtta      3968

acttagcttg aaggatctgt ttttaaaaat cataaactgt gtgcagactc aataaaatca      4028

tgtacatttc tgaaatgacc tcaagatgtc ctccttgttc tactcatata tatctatctt      4088

atatacttac tattttactt ctagagatag tacataaagg tggtatgtgt gtgtatgcta      4148

ctacaaaaaa gttgttaact aaattaacat tgggaaatct tatattccat atattagcat      4208

ttagtccaat gtctttttaa gcttatttaa ttaaaaaatt tccagtgagc ttatcatgct      4268

gtctttacat ggggttttca attttgcatg ctcgattatt ccctgtacaa tatttaaaat      4328

ttattgcttg atacttttga caacaaatta ggttttgtac aattgaactt aaataaatgt      4388

cattaaaata aataaatgca atatgtatta atattcattg tataaaaata gaagaataca      4448

aacatatttg ttaaatattt acatatgaaa tttaatatag ctatttttat ggaatttttc      4508

attgatatga aaaatatgat attgcatatg catagttccc atgttaaatc ccattcataa      4568

ctttcattaa agcatttact ttgaatttc                                    4597

<210>  8
<211>  1143
<212>  PRT
<213>  Homo sapiens

<400>  8

Met Tyr Leu Trp Leu Lys Leu Leu Ala Phe Gly Phe Ala Phe Leu Asp
1               5                   10                  15

Thr Glu Val Phe Val Thr Gly Gln Ser Pro Thr Pro Ser Pro Thr Asp
            20                  25                  30

Ala Tyr Leu Asn Ala Ser Glu Thr Thr Thr Leu Ser Pro Ser Gly Ser
        35                  40                  45

Ala Val Ile Ser Thr Thr Thr Ile Ala Thr Thr Pro Ser Lys Pro Thr
    50                  55                  60

Cys Asp Glu Lys Tyr Ala Asn Ile Thr Val Asp Tyr Leu Tyr Asn Lys
65                  70                  75                  80

Glu Thr Lys Leu Phe Thr Ala Lys Leu Asn Val Asn Glu Asn Val Glu
                85                  90                  95

Cys Gly Asn Asn Thr Cys Thr Asn Asn Glu Val His Asn Leu Thr Glu
            100                 105                 110

Cys Lys Asn Ala Ser Val Ser Ile Ser His Asn Ser Cys Thr Ala Pro
        115                 120                 125

Asp Lys Thr Leu Ile Leu Asp Val Pro Pro Gly Val Glu Lys Phe Gln
    130                 135                 140

Leu His Asp Cys Thr Gln Val Glu Lys Ala Asp Thr Thr Ile Cys Leu
145                 150                 155                 160

Lys Trp Lys Asn Ile Glu Thr Phe Thr Cys Asp Thr Gln Asn Ile Thr
                165                 170                 175

Tyr Arg Phe Gln Cys Gly Asn Met Ile Phe Asp Asn Lys Glu Ile Lys
            180                 185                 190

Leu Glu Asn Leu Glu Pro Glu His Glu Tyr Lys Cys Asp Ser Glu Ile
        195                 200                 205

Leu Tyr Asn Asn His Lys Phe Thr Asn Ala Ser Lys Ile Ile Lys Thr
    210                 215                 220

Asp Phe Gly Ser Pro Gly Glu Pro Gln Ile Ile Phe Cys Arg Ser Glu
225                 230                 235                 240

Ala Ala His Gln Gly Val Ile Thr Trp Asn Pro Pro Gln Arg Ser Phe

                245                 250                 255

His Asn Phe Thr Leu Cys Tyr Ile Lys Glu Thr Glu Lys Asp Cys Leu
            260                 265                 270

Asn Leu Asp Lys Asn Leu Ile Lys Tyr Asp Leu Gln Asn Leu Lys Pro
        275                 280                 285

Tyr Thr Lys Tyr Val Leu Ser Leu His Ala Tyr Ile Ile Ala Lys Val
    290                 295                 300

Gln Arg Asn Gly Ser Ala Ala Met Cys His Phe Thr Thr Lys Ser Ala
305                 310                 315                 320

Pro Pro Ser Gln Val Trp Asn Met Thr Val Ser Met Thr Ser Asp Asn
                325                 330                 335

Ser Met His Val Lys Cys Arg Pro Pro Arg Asp Arg Asn Gly Pro His
            340                 345                 350

Glu Arg Tyr His Leu Glu Val Glu Ala Gly Asn Thr Leu Val Arg Asn
        355                 360                 365

Glu Ser His Lys Asn Cys Asp Phe Arg Val Lys Asp Leu Gln Tyr Ser
    370                 375                 380

Thr Asp Tyr Thr Phe Lys Ala Tyr Phe His Asn Gly Asp Tyr Pro Gly
385                 390                 395                 400

Glu Pro Phe Ile Leu His His Ser Thr Ser Tyr Asn Ser Lys Ala Leu
                405                 410                 415

Ile Ala Phe Leu Ala Phe Leu Ile Ile Val Thr Ser Ile Ala Leu Leu
            420                 425                 430

Val Val Leu Tyr Lys Ile Tyr Asp Leu His Lys Lys Arg Ser Cys Asn
        435                 440                 445

Leu Asp Glu Gln Gln Glu Leu Val Glu Arg Asp Asp Glu Lys Gln Leu
    450                 455                 460

Met Asn Val Glu Pro Ile His Ala Asp Ile Leu Leu Glu Thr Tyr Lys
465                 470                 475                 480

Arg Lys Ile Ala Asp Glu Gly Arg Leu Phe Leu Ala Glu Phe Gln Ser
                485                 490                 495

Ile Pro Arg Val Phe Ser Lys Phe Pro Ile Lys Glu Ala Arg Lys Pro
            500                 505                 510

Phe Asn Gln Asn Lys Asn Arg Tyr Val Asp Ile Leu Pro Tyr Asp Tyr
        515                 520                 525

Asn Arg Val Glu Leu Ser Glu Ile Asn Gly Asp Ala Gly Ser Asn Tyr
    530                 535                 540

Ile Asn Ala Ser Tyr Ile Asp Gly Phe Lys Glu Pro Arg Lys Tyr Ile
545                 550                 555                 560

Ala Ala Gln Gly Pro Arg Asp Glu Thr Val Asp Asp Phe Trp Arg Met
                565                 570                 575

Ile Trp Glu Gln Lys Ala Thr Val Ile Val Met Val Thr Arg Cys Glu
            580                 585                 590

Glu Gly Asn Arg Asn Lys Cys Ala Glu Tyr Trp Pro Ser Met Glu Glu
        595                 600                 605

Gly Thr Arg Ala Phe Gly Asp Val Val Val Lys Ile Asn Gln His Lys
    610                 615                 620

Arg Cys Pro Asp Tyr Ile Ile Gln Lys Leu Asn Ile Val Asn Lys Lys
625                 630                 635                 640

Glu Lys Ala Thr Gly Arg Glu Val Thr His Ile Gln Phe Thr Ser Trp
                645                 650                 655

Pro Asp His Gly Val Pro Glu Asp Pro His Leu Leu Leu Lys Leu Arg
            660                 665                 670

Arg Arg Val Asn Ala Phe Ser Asn Phe Phe Ser Gly Pro Ile Val Val
        675                 680                 685

His Cys Ser Ala Gly Val Gly Arg Thr Gly Thr Tyr Ile Gly Ile Asp
    690                 695                 700

Ala Met Leu Glu Gly Leu Glu Ala Glu Asn Lys Val Asp Val Tyr Gly
705                 710                 715                 720

Tyr Val Val Lys Leu Arg Arg Gln Arg Cys Leu Met Val Gln Val Glu
                725                 730                 735

Ala Gln Tyr Ile Leu Ile His Gln Ala Leu Val Glu Tyr Asn Gln Phe
            740                 745                 750

Gly Glu Thr Glu Val Asn Leu Ser Glu Leu His Pro Tyr Leu His Asn
        755                 760                 765

Met Lys Lys Arg Asp Pro Pro Ser Glu Pro Ser Pro Leu Glu Ala Glu
    770                 775                 780

Phe Gln Arg Leu Pro Ser Tyr Arg Ser Trp Arg Thr Gln His Ile Gly
785                 790                 795                 800

Asn Gln Glu Glu Asn Lys Ser Lys Asn Arg Asn Ser Asn Val Ile Pro
                805                 810                 815

Tyr Asp Tyr Asn Arg Val Pro Leu Lys His Glu Leu Glu Met Ser Lys
            820                 825                 830

Glu Ser Glu His Asp Ser Asp Glu Ser Ser Asp Asp Asp Ser Asp Ser
        835                 840                 845

Glu Glu Pro Ser Lys Tyr Ile Asn Ala Ser Phe Ile Met Ser Tyr Trp
    850                 855                 860

Lys Pro Glu Val Met Ile Ala Ala Gln Gly Pro Leu Lys Glu Thr Ile
865                 870                 875                 880

Gly Asp Phe Trp Gln Met Ile Phe Gln Arg Lys Val Lys Val Ile Val
                885                 890                 895

Met Leu Thr Glu Leu Lys His Gly Asp Gln Glu Ile Cys Ala Gln Tyr
            900                 905                 910

Trp Gly Glu Gly Lys Gln Thr Tyr Gly Asp Ile Glu Val Asp Leu Lys
        915                 920                 925

Asp Thr Asp Lys Ser Ser Thr Tyr Thr Leu Arg Val Phe Glu Leu Arg
    930                 935                 940

His Ser Lys Arg Lys Asp Ser Arg Thr Val Tyr Gln Tyr Gln Tyr Thr
945                 950                 955                 960

Asn Trp Ser Val Glu Gln Leu Pro Ala Glu Pro Lys Glu Leu Ile Ser
                965                 970                 975

Met Ile Gln Val Val Lys Gln Lys Leu Pro Gln Lys Asn Ser Ser Glu
            980                 985                 990

Gly Asn Lys His His Lys Ser Thr  Pro Leu Leu Ile His  Cys Arg Asp
        995                 1000                1005

Gly Ser  Gln Gln Thr Gly Ile  Phe Cys Ala Leu Leu  Asn Leu Leu
    1010                 1015                1020

Glu Ser  Ala Glu Thr Glu Glu  Val Val Asp Ile Phe  Gln Val Val
    1025                 1030                1035

Lys Ala  Leu Arg Lys Ala Arg  Pro Gly Met Val Ser  Thr Phe Glu
    1040                 1045                1050

Gln Tyr  Gln Phe Leu Tyr Asp  Val Ile Ala Ser Thr  Tyr Pro Ala
    1055                 1060                1065

Gln Asn Gly Gln Val Lys Lys Asn Asn His Gln Glu Asp Lys Ile
    1070                1075                1080

Glu Phe Asp Asn Glu Val Asp Lys Val Lys Gln Asp Ala Asn Cys
    1085                1090                1095

Val Asn Pro Leu Gly Ala Pro Glu Lys Leu Pro Glu Ala Lys Glu
    1100                1105                1110

Gln Ala Glu Gly Ser Glu Pro Thr Ser Gly Thr Glu Gly Pro Glu
    1115                1120                1125

His Ser Val Asn Gly Pro Ala Ser Pro Ala Leu Asn Gln Gly Ser
    1130                1135                1140

<210>  9
<211>  1528
<212>  DNA
<213>  Homo sapiens

<220>
<221>  CDS
<222>  (179)..(1123)
<223>  EpCAM

<400>  9
cggcgagcga gcaccttcga cgcggtccgg ggaccccctc gtcgctgtcc tcccgacgcg      60

gacccgcgtg ccccaggcct cgcgctgccc ggccggctcc tcgtgtccca ctcccggcgc     120

acgccctccc gcgagtcccg ggcccctccc gcgcccctct tctcggcgcg cgcgcagc       178

atg gcg ccc ccg cag gtc ctc gcg ttc ggg ctt ctg ctt gcc gcg gcg       226
Met Ala Pro Pro Gln Val Leu Ala Phe Gly Leu Leu Leu Ala Ala Ala
1               5                   10                  15

acg gcg act ttt gcc gca gct cag gaa gaa tgt gtc tgt gaa aac tac       274
Thr Ala Thr Phe Ala Ala Ala Gln Glu Glu Cys Val Cys Glu Asn Tyr
            20                  25                  30

aag ctg gcc gta aac tgc ttt gtg aat aat aat cgt caa tgc cag tgt       322
Lys Leu Ala Val Asn Cys Phe Val Asn Asn Asn Arg Gln Cys Gln Cys
        35                  40                  45

act tca gtt ggt gca caa aat act gtc att tgc tca aag ctg gct gcc       370
Thr Ser Val Gly Ala Gln Asn Thr Val Ile Cys Ser Lys Leu Ala Ala
    50                  55                  60

aaa tgt ttg gtg atg aag gca gaa atg aat ggc tca aaa ctt ggg aga       418
Lys Cys Leu Val Met Lys Ala Glu Met Asn Gly Ser Lys Leu Gly Arg
65                  70                  75                  80

aga gca aaa cct gaa ggg gcc ctc cag aac aat gat ggg ctt tat gat       466
Arg Ala Lys Pro Glu Gly Ala Leu Gln Asn Asn Asp Gly Leu Tyr Asp
                85                  90                  95

cct gac tgc gat gag agc ggg ctc ttt aag gcc aag cag tgc aac ggc       514
Pro Asp Cys Asp Glu Ser Gly Leu Phe Lys Ala Lys Gln Cys Asn Gly
            100                 105                 110

acc tcc acg tgc tgg tgt gtg aac act gct ggg gtc aga aga aca gac       562
Thr Ser Thr Cys Trp Cys Val Asn Thr Ala Gly Val Arg Arg Thr Asp
        115                 120                 125

aag gac act gaa ata acc tgc tct gag cga gtg aga acc tac tgg atc     610
Lys Asp Thr Glu Ile Thr Cys Ser Glu Arg Val Arg Thr Tyr Trp Ile
    130                 135                 140

atc att gaa cta aaa cac aaa gca aga gaa aaa cct tat gat agt aaa     658
Ile Ile Glu Leu Lys His Lys Ala Arg Glu Lys Pro Tyr Asp Ser Lys
145                 150                 155                 160

agt ttg cgg act gca ctt cag aag gag atc aca acg cgt tat caa ctg     706
Ser Leu Arg Thr Ala Leu Gln Lys Glu Ile Thr Thr Arg Tyr Gln Leu
                165                 170                 175

gat cca aaa ttt atc acg agt att ttg tat gag aat aat gtt atc act     754
Asp Pro Lys Phe Ile Thr Ser Ile Leu Tyr Glu Asn Asn Val Ile Thr
            180                 185                 190

att gat ctg gtt caa aat tct tct caa aaa act cag aat gat gtg gac     802
Ile Asp Leu Val Gln Asn Ser Ser Gln Lys Thr Gln Asn Asp Val Asp
        195                 200                 205

ata gct gat gtg gct tat tat ttt gaa aaa gat gtt aaa ggt gaa tcc     850
Ile Ala Asp Val Ala Tyr Tyr Phe Glu Lys Asp Val Lys Gly Glu Ser
    210                 215                 220

ttg ttt cat tct aag aaa atg gac ctg aca gta aat ggg gaa caa ctg     898
Leu Phe His Ser Lys Lys Met Asp Leu Thr Val Asn Gly Glu Gln Leu
225                 230                 235                 240

gat ctg gat cct ggt caa act tta att tat tat gtt gat gaa aaa gca     946
Asp Leu Asp Pro Gly Gln Thr Leu Ile Tyr Tyr Val Asp Glu Lys Ala
                245                 250                 255

cct gaa ttc tca atg cag ggt cta aaa gct ggt gtt att gct gtt att     994
Pro Glu Phe Ser Met Gln Gly Leu Lys Ala Gly Val Ile Ala Val Ile
            260                 265                 270

gtg gtt gtg gtg ata gca gtt gtt gct gga att gtt gtg ctg gtt att     1042
Val Val Val Val Ile Ala Val Val Ala Gly Ile Val Val Leu Val Ile
        275                 280                 285

tcc aga aag aag aga atg gca aag tat gag aag gct gag ata aag gag     1090
Ser Arg Lys Lys Arg Met Ala Lys Tyr Glu Lys Ala Glu Ile Lys Glu
    290                 295                 300

atg ggt gag atg cat agg gaa ctc aat gca taa ctatataatt tgaagattat     1143
Met Gly Glu Met His Arg Glu Leu Asn Ala
305                 310

agaagaaggg aaatagcaaa tggacacaaa ttacaaatgt gtgtgcgtgg gacgaagaca     1203

tctttgaagg tcatgagttt gttagtttaa catcatatat ttgtaatagt gaaacctgta     1263

ctcaaaatat aagcagcttg aaactggctt taccaatctt gaaatttgac cacaagtgtc     1323

ttatatatgc agatctaatg taaaatccag aacttggact ccatcgttaa aattatttat     1383

gtgtaacatt caaatgtgtg cattaaatat gcttccacag taaaatctga aaaactgatt     1443

tgtgattgaa agctgccttt ctatttactt gagtcttgta catacatact tttttatgag     1503

ctatgaaata aaacatttta aactg     1528

<210>  10
<211>  314
<212>  PRT
<213>  Homo sapiens

<400>  10

Met Ala Pro Pro Gln Val Leu Ala Phe Gly Leu Leu Leu Ala Ala Ala
1               5                   10                  15

Thr Ala Thr Phe Ala Ala Ala Gln Glu Glu Cys Val Cys Glu Asn Tyr
            20                  25                  30

Lys Leu Ala Val Asn Cys Phe Val Asn Asn Asn Arg Gln Cys Gln Cys
        35                  40                  45

Thr Ser Val Gly Ala Gln Asn Thr Val Ile Cys Ser Lys Leu Ala Ala
    50                  55                  60

Lys Cys Leu Val Met Lys Ala Glu Met Asn Gly Ser Lys Leu Gly Arg
65                  70                  75                  80

Arg Ala Lys Pro Glu Gly Ala Leu Gln Asn Asn Asp Gly Leu Tyr Asp
                85                  90                  95

Pro Asp Cys Asp Glu Ser Gly Leu Phe Lys Ala Lys Gln Cys Asn Gly
            100                 105                 110

Thr Ser Thr Cys Trp Cys Val Asn Thr Ala Gly Val Arg Arg Thr Asp
        115                 120                 125

Lys Asp Thr Glu Ile Thr Cys Ser Glu Arg Val Arg Thr Tyr Trp Ile
    130                 135                 140

Ile Ile Glu Leu Lys His Lys Ala Arg Glu Lys Pro Tyr Asp Ser Lys
145                 150                 155                 160

Ser Leu Arg Thr Ala Leu Gln Lys Glu Ile Thr Thr Arg Tyr Gln Leu
                165                 170                 175

Asp Pro Lys Phe Ile Thr Ser Ile Leu Tyr Glu Asn Asn Val Ile Thr
            180                 185                 190

Ile Asp Leu Val Gln Asn Ser Ser Gln Lys Thr Gln Asn Asp Val Asp
        195                 200                 205

Ile Ala Asp Val Ala Tyr Tyr Phe Glu Lys Asp Val Lys Gly Glu Ser
    210                 215                 220

Leu Phe His Ser Lys Lys Met Asp Leu Thr Val Asn Gly Glu Gln Leu
225                 230                 235                 240

Asp Leu Asp Pro Gly Gln Thr Leu Ile Tyr Tyr Val Asp Glu Lys Ala
                245                 250                 255

Pro Glu Phe Ser Met Gln Gly Leu Lys Ala Gly Val Ile Ala Val Ile
            260                 265                 270

Val Val Val Val Ile Ala Val Val Ala Gly Ile Val Val Leu Val Ile
        275                 280                 285

Ser Arg Lys Lys Arg Met Ala Lys Tyr Glu Lys Ala Glu Ile Lys Glu
    290                 295                 300

Met Gly Glu Met His Arg Glu Leu Asn Ala
305                 310

<210>  11
<211>  4530
<212>  DNA
<213>  Homo sapiens

<220>
<221>  CDS
<222>  (151)..(3918)
<223>  Her2

<400>  11
aattctcgag ctcgtcgacc ggtcgacgag ctcgagggtc gacgagctcg agggcgcgcg      60

cccggccccc acccctcgca gcaccccgcg ccccgcgccc tcccagccgg gtccagccgg     120

agccatgggg ccggagccgc agtgagcacc atg gag ctg gcg gcc ttg tgc cgc      174
                                 Met Glu Leu Ala Ala Leu Cys Arg
                                 1               5

tgg ggg ctc ctc ctc gcc ctc ttg ccc ccc gga gcc gcg agc acc caa      222
Trp Gly Leu Leu Leu Ala Leu Leu Pro Pro Gly Ala Ala Ser Thr Gln
    10                  15                  20

gtg tgc acc ggc aca gac atg aag ctg cgg ctc cct gcc agt ccc gag      270
Val Cys Thr Gly Thr Asp Met Lys Leu Arg Leu Pro Ala Ser Pro Glu
25                  30                  35                  40

acc cac ctg gac atg ctc cgc cac ctc tac cag ggc tgc cag gtg gtg      318
Thr His Leu Asp Met Leu Arg His Leu Tyr Gln Gly Cys Gln Val Val
                45                  50                  55

cag gga aac ctg gaa ctc acc tac ctg ccc acc aat gcc agc ctg tcc      366
Gln Gly Asn Leu Glu Leu Thr Tyr Leu Pro Thr Asn Ala Ser Leu Ser
            60                  65                  70

ttc ctg cag gat atc cag gag gtg cag ggc tac gtg ctc atc gct cac      414
Phe Leu Gln Asp Ile Gln Glu Val Gln Gly Tyr Val Leu Ile Ala His
        75                  80                  85

aac caa gtg agg cag gtc cca ctg cag agg ctg cgg att gtg cga ggc      462
Asn Gln Val Arg Gln Val Pro Leu Gln Arg Leu Arg Ile Val Arg Gly
    90                  95                  100

acc cag ctc ttt gag gac aac tat gcc ctg gcc gtg cta gac aat gga      510
Thr Gln Leu Phe Glu Asp Asn Tyr Ala Leu Ala Val Leu Asp Asn Gly
105                 110                 115                 120

gac ccg ctg aac aat acc acc cct gtc aca ggg gcc tcc cca gga ggc      558
Asp Pro Leu Asn Asn Thr Thr Pro Val Thr Gly Ala Ser Pro Gly Gly
                125                 130                 135

ctg cgg gag ctg cag ctt cga agc ctc aca gag atc ttg aaa gga ggg      606
Leu Arg Glu Leu Gln Leu Arg Ser Leu Thr Glu Ile Leu Lys Gly Gly
            140                 145                 150

gtc ttg atc cag cgg aac ccc cag ctc tgc tac cag gac acg att ttg 654
Val Leu Ile Gln Arg Asn Pro Gln Leu Cys Tyr Gln Asp Thr Ile Leu
        155                 160                 165

tgg aag gac atc ttc cac aag aac aac cag ctg gct ctc aca ctg ata 702
Trp Lys Asp Ile Phe His Lys Asn Asn Gln Leu Ala Leu Thr Leu Ile
    170                 175                 180

gac acc aac cgc tct cgg gcc tgc cac ccc tgt tct ccg atg tgt aag 750
Asp Thr Asn Arg Ser Arg Ala Cys His Pro Cys Ser Pro Met Cys Lys
185                 190                 195                 200

ggc tcc cgc tgc tgg gga gag agt tct gag gat tgt cag agc ctg acg 798
Gly Ser Arg Cys Trp Gly Glu Ser Ser Glu Asp Cys Gln Ser Leu Thr
                205                 210                 215

cgc act gtc tgt gcc ggt ggc tgt gcc cgc tgc aag ggg cca ctg ccc 846
Arg Thr Val Cys Ala Gly Gly Cys Ala Arg Cys Lys Gly Pro Leu Pro
            220                 225                 230

act gac tgc tgc cat gag cag tgt gct gcc ggc tgc acg ggc ccc aag 894
Thr Asp Cys Cys His Glu Gln Cys Ala Ala Gly Cys Thr Gly Pro Lys
        235                 240                 245

cac tct gac tgc ctg gcc tgc ctc cac ttc aac cac agt ggc atc tgt 942
His Ser Asp Cys Leu Ala Cys Leu His Phe Asn His Ser Gly Ile Cys
    250                 255                 260

gag ctg cac tgc cca gcc ctg gtc acc tac aac aca gac acg ttt gag 990
Glu Leu His Cys Pro Ala Leu Val Thr Tyr Asn Thr Asp Thr Phe Glu
265                 270                 275                 280

tcc atg ccc aat ccc gag ggc cgg tat aca ttc ggc gcc agc tgt gtg 1038
Ser Met Pro Asn Pro Glu Gly Arg Tyr Thr Phe Gly Ala Ser Cys Val
                285                 290                 295

act gcc tgt ccc tac aac tac ctt tct acg gac gtg gga tcc tgc acc 1086
Thr Ala Cys Pro Tyr Asn Tyr Leu Ser Thr Asp Val Gly Ser Cys Thr
            300                 305                 310

ctc gtc tgc ccc ctg cac aac caa gag gtg aca gca gag gat gga aca 1134
Leu Val Cys Pro Leu His Asn Gln Glu Val Thr Ala Glu Asp Gly Thr
        315                 320                 325

cag cgg tgt gag aag tgc agc aag ccc tgt gcc cga gtg tgc tat ggt 1182
Gln Arg Cys Glu Lys Cys Ser Lys Pro Cys Ala Arg Val Cys Tyr Gly
    330                 335                 340

ctg ggc atg gag cac ttg cga gag gtg agg gca gtt acc agt gcc aat 1230
Leu Gly Met Glu His Leu Arg Glu Val Arg Ala Val Thr Ser Ala Asn
345                 350                 355                 360

atc cag gag ttt gct ggc tgc aag aag atc ttt ggg agc ctg gca ttt 1278
Ile Gln Glu Phe Ala Gly Cys Lys Lys Ile Phe Gly Ser Leu Ala Phe
                365                 370                 375

ctg ccg gag agc ttt gat ggg gac cca gcc tcc aac act gcc ccg ctc 1326
Leu Pro Glu Ser Phe Asp Gly Asp Pro Ala Ser Asn Thr Ala Pro Leu
            380                 385                 390

cag cca gag cag ctc caa gtg ttt gag act ctg gaa gag atc aca ggt 1374
Gln Pro Glu Gln Leu Gln Val Phe Glu Thr Leu Glu Glu Ile Thr Gly
        395                 400                 405

tac cta tac atc tca gca tgg ccg gac agc ctg cct gac ctc agc gtc 1422
Tyr Leu Tyr Ile Ser Ala Trp Pro Asp Ser Leu Pro Asp Leu Ser Val
    410                 415                 420

ttc cag aac ctg caa gta atc cgg gga cga att ctg cac aat ggc gcc 1470

Phe Gln Asn Leu Gln Val Ile Arg Gly Arg Ile Leu His Asn Gly Ala
425                 430                 435                 440

tac tcg ctg acc ctg caa ggg ctg ggc atc agc tgg ctg ggg ctg cgc      1518
Tyr Ser Leu Thr Leu Gln Gly Leu Gly Ile Ser Trp Leu Gly Leu Arg
                445                 450                 455

tca ctg agg gaa ctg ggc agt gga ctg gcc ctc atc cac cat aac acc      1566
Ser Leu Arg Glu Leu Gly Ser Gly Leu Ala Leu Ile His His Asn Thr
            460                 465                 470

cac ctc tgc ttc gtg cac acg gtg ccc tgg gac cag ctc ttt cgg aac      1614
His Leu Cys Phe Val His Thr Val Pro Trp Asp Gln Leu Phe Arg Asn
        475                 480                 485

ccg cac caa gct ctg ctc cac act gcc aac cgg cca gag gac gag tgt      1662
Pro His Gln Ala Leu Leu His Thr Ala Asn Arg Pro Glu Asp Glu Cys
    490                 495                 500

gtg ggc gag ggc ctg gcc tgc cac cag ctg tgc gcc cga ggg cac tgc      1710
Val Gly Glu Gly Leu Ala Cys His Gln Leu Cys Ala Arg Gly His Cys
505                 510                 515                 520

tgg ggt cca ggg ccc acc cag tgt gtc aac tgc agc cag ttc ctt cgg      1758
Trp Gly Pro Gly Pro Thr Gln Cys Val Asn Cys Ser Gln Phe Leu Arg
                525                 530                 535

ggc cag gag tgc gtg gag gaa tgc cga gta ctg cag ggg ctc ccc agg      1806
Gly Gln Glu Cys Val Glu Glu Cys Arg Val Leu Gln Gly Leu Pro Arg
            540                 545                 550

gag tat gtg aat gcc agg cac tgt ttg ccg tgc cac cct gag tgt cag      1854
Glu Tyr Val Asn Ala Arg His Cys Leu Pro Cys His Pro Glu Cys Gln
        555                 560                 565

ccc cag aat ggc tca gtg acc tgt ttt gga ccg gag gct gac cag tgt      1902
Pro Gln Asn Gly Ser Val Thr Cys Phe Gly Pro Glu Ala Asp Gln Cys
    570                 575                 580

gtg gcc tgt gcc cac tat aag gac cct ccc ttc tgc gtg gcc cgc tgc      1950
Val Ala Cys Ala His Tyr Lys Asp Pro Pro Phe Cys Val Ala Arg Cys
585                 590                 595                 600

ccc agc ggt gtg aaa cct gac ctc tcc tac atg ccc atc tgg aag ttt      1998
Pro Ser Gly Val Lys Pro Asp Leu Ser Tyr Met Pro Ile Trp Lys Phe
                605                 610                 615

cca gat gag gag ggc gca tgc cag cct tgc ccc atc aac tgc acc cac      2046
Pro Asp Glu Glu Gly Ala Cys Gln Pro Cys Pro Ile Asn Cys Thr His
            620                 625                 630

tcc tgt gtg gac ctg gat gac aag ggc tgc ccc gcc gag cag aga gcc      2094
Ser Cys Val Asp Leu Asp Asp Lys Gly Cys Pro Ala Glu Gln Arg Ala
        635                 640                 645

agc cct ctg acg tcc atc gtc tct gcg gtg gtt ggc att ctg ctg gtc      2142
Ser Pro Leu Thr Ser Ile Val Ser Ala Val Val Gly Ile Leu Leu Val
    650                 655                 660

gtg gtc ttg ggg gtg gtc ttt ggg atc ctc atc aag cga cgg cag cag      2190
Val Val Leu Gly Val Val Phe Gly Ile Leu Ile Lys Arg Arg Gln Gln
665                 670                 675                 680

aag atc cgg aag tac acg atg cgg aga ctg ctg cag gaa acg gag ctg      2238
Lys Ile Arg Lys Tyr Thr Met Arg Arg Leu Leu Gln Glu Thr Glu Leu
                685                 690                 695

gtg gag ccg ctg aca cct agc gga gcg atg ccc aac cag gcg cag atg      2286
Val Glu Pro Leu Thr Pro Ser Gly Ala Met Pro Asn Gln Ala Gln Met

                    700                 705                 710

cgg atc ctg aaa gag acg gag ctg agg aag gtg aag gtg ctt gga tct      2334
Arg Ile Leu Lys Glu Thr Glu Leu Arg Lys Val Lys Val Leu Gly Ser
            715                 720                 725

ggc gct ttt ggc aca gtc tac aag ggc atc tgg atc cct gat ggg gag      2382
Gly Ala Phe Gly Thr Val Tyr Lys Gly Ile Trp Ile Pro Asp Gly Glu
    730                 735                 740

aat gtg aaa att cca gtg gcc atc aaa gtg ttg agg gaa aac aca tcc      2430
Asn Val Lys Ile Pro Val Ala Ile Lys Val Leu Arg Glu Asn Thr Ser
745                 750                 755                 760

ccc aaa gcc aac aaa gaa atc tta gac gaa gca tac gtg atg gct ggt      2478
Pro Lys Ala Asn Lys Glu Ile Leu Asp Glu Ala Tyr Val Met Ala Gly
                765                 770                 775

gtg ggc tcc cca tat gtc tcc cgc ctt ctg ggc atc tgc ctg aca tcc      2526
Val Gly Ser Pro Tyr Val Ser Arg Leu Leu Gly Ile Cys Leu Thr Ser
            780                 785                 790

acg gtg cag ctg gtg aca cag ctt atg ccc tat ggc tgc ctc tta gac      2574
Thr Val Gln Leu Val Thr Gln Leu Met Pro Tyr Gly Cys Leu Leu Asp
        795                 800                 805

cat gtc cgg gaa aac cgc gga cgc ctg ggc tcc cag gac ctg ctg aac      2622
His Val Arg Glu Asn Arg Gly Arg Leu Gly Ser Gln Asp Leu Leu Asn
    810                 815                 820

tgg tgt atg cag att gcc aag ggg atg agc tac ctg gag gat gtg cgg      2670
Trp Cys Met Gln Ile Ala Lys Gly Met Ser Tyr Leu Glu Asp Val Arg
825                 830                 835                 840

ctc gta cac agg gac ttg gcc gct cgg aac gtg ctg gtc aag agt ccc      2718
Leu Val His Arg Asp Leu Ala Ala Arg Asn Val Leu Val Lys Ser Pro
                845                 850                 855

aac cat gtc aaa att aca gac ttc ggg ctg gct cgg ctg ctg gac att      2766
Asn His Val Lys Ile Thr Asp Phe Gly Leu Ala Arg Leu Leu Asp Ile
            860                 865                 870

gac gag aca gag tac cat gca gat ggg ggc aag gtg ccc atc aag tgg      2814
Asp Glu Thr Glu Tyr His Ala Asp Gly Gly Lys Val Pro Ile Lys Trp
        875                 880                 885

atg gcg ctg gag tcc att ctc cgc cgg cgg ttc acc cac cag agt gat      2862
Met Ala Leu Glu Ser Ile Leu Arg Arg Arg Phe Thr His Gln Ser Asp
    890                 895                 900

gtg tgg agt tat ggt gtg act gtg tgg gag ctg atg act ttt ggg gcc      2910
Val Trp Ser Tyr Gly Val Thr Val Trp Glu Leu Met Thr Phe Gly Ala
905                 910                 915                 920

aaa cct tac gat ggg atc cca gcc cgg gag atc cct gac ctg ctg gaa      2958
Lys Pro Tyr Asp Gly Ile Pro Ala Arg Glu Ile Pro Asp Leu Leu Glu
                925                 930                 935

aag ggg gag cgg ctg ccc cag ccc ccc atc tgc acc att gat gtc tac      3006
Lys Gly Glu Arg Leu Pro Gln Pro Pro Ile Cys Thr Ile Asp Val Tyr
            940                 945                 950

atg atc atg gtc aaa tgt tgg atg att gac tct gaa tgt cgg cca aga      3054
Met Ile Met Val Lys Cys Trp Met Ile Asp Ser Glu Cys Arg Pro Arg
        955                 960                 965

ttc cgg gag ttg gtg tct gaa ttc tcc cgc atg gcc agg gac ccc cag      3102
Phe Arg Glu Leu Val Ser Glu Phe Ser Arg Met Ala Arg Asp Pro Gln
    970                 975                 980

cgc ttt gtg gtc atc cag aat gag gac ttg ggc cca gcc agt ccc ttg 3150
Arg Phe Val Val Ile Gln Asn Glu Asp Leu Gly Pro Ala Ser Pro Leu
985                 990                 995                 1000

gac agc acc ttc tac cgc tca ctg ctg gag gac gat gac atg ggg 3195
Asp Ser Thr Phe Tyr Arg Ser Leu Leu Glu Asp Asp Asp Met Gly
                1005                1010                1015

gac ctg gtg gat gct gag gag tat ctg gta ccc cag cag ggc ttc 3240
Asp Leu Val Asp Ala Glu Glu Tyr Leu Val Pro Gln Gln Gly Phe
                1020                1025                1030

ttc tgt cca gac cct gcc ccg ggc gct ggg ggc atg gtc cac cac 3285
Phe Cys Pro Asp Pro Ala Pro Gly Ala Gly Gly Met Val His His
                1035                1040                1045

agg cac cgc agc tca tct acc agg agt ggc ggt ggg gac ctg aca 3330
Arg His Arg Ser Ser Ser Thr Arg Ser Gly Gly Gly Asp Leu Thr
                1050                1055                1060

cta ggg ctg gag ccc tct gaa gag gag gcc ccc agg tct cca ctg 3375
Leu Gly Leu Glu Pro Ser Glu Glu Glu Ala Pro Arg Ser Pro Leu
                1065                1070                1075

gca ccc tcc gaa ggg gct ggc tcc gat gta ttt gat ggt gac ctg 3420
Ala Pro Ser Glu Gly Ala Gly Ser Asp Val Phe Asp Gly Asp Leu
                1080                1085                1090

gga atg ggg gca gcc aag ggg ctg caa agc ctc ccc aca cat gac 3465
Gly Met Gly Ala Ala Lys Gly Leu Gln Ser Leu Pro Thr His Asp
                1095                1100                1105

ccc agc cct cta cag cgg tac agt gag gac ccc aca gta ccc ctg 3510
Pro Ser Pro Leu Gln Arg Tyr Ser Glu Asp Pro Thr Val Pro Leu
                1110                1115                1120

ccc tct gag act gat ggc tac gtt gcc ccc ctg acc tgc agc ccc 3555
Pro Ser Glu Thr Asp Gly Tyr Val Ala Pro Leu Thr Cys Ser Pro
                1125                1130                1135

cag cct gaa tat gtg aac cag cca gat gtt cgg ccc cag ccc cct 3600
Gln Pro Glu Tyr Val Asn Gln Pro Asp Val Arg Pro Gln Pro Pro
                1140                1145                1150

tcg ccc cga gag ggc cct ctg cct gct gcc cga cct gct ggt gcc 3645
Ser Pro Arg Glu Gly Pro Leu Pro Ala Ala Arg Pro Ala Gly Ala
                1155                1160                1165

act ctg gaa agg gcc aag act ctc tcc cca ggg aag aat ggg gtc 3690
Thr Leu Glu Arg Ala Lys Thr Leu Ser Pro Gly Lys Asn Gly Val
                1170                1175                1180

gtc aaa gac gtt ttt gcc ttt ggg ggt gcc gtg gag aac ccc gag 3735
Val Lys Asp Val Phe Ala Phe Gly Gly Ala Val Glu Asn Pro Glu
                1185                1190                1195

tac ttg aca ccc cag gga gga gct gcc cct cag ccc cac cct cct 3780
Tyr Leu Thr Pro Gln Gly Gly Ala Ala Pro Gln Pro His Pro Pro
                1200                1205                1210

cct gcc ttc agc cca gcc ttc gac aac ctc tat tac tgg gac cag 3825
Pro Ala Phe Ser Pro Ala Phe Asp Asn Leu Tyr Tyr Trp Asp Gln
                1215                1220                1225

gac cca cca gag cgg ggg gct cca ccc agc acc ttc aaa ggg aca 3870
Asp Pro Pro Glu Arg Gly Ala Pro Pro Ser Thr Phe Lys Gly Thr
                1230                1235                1240

cct acg gca gag aac  cca gag tac ctg ggt  ctg gac gtg cca gtg       3915
Pro Thr Ala Glu Asn  Pro Glu Tyr Leu Gly  Leu Asp Val Pro Val
                1245                 1250                 1255

tga accagaaggc caagtccgca gaagccctga tgtgtcctca gggagcaggg          3968

aaggcctgac ttctgctggc atcaagaggt gggagggccc tccgaccact tccaggggaa   4028

cctgccatgc caggaacctg tcctaaggaa ccttccttcc tgcttgagtt cccagatggc   4088

tggaaggggt ccagcctcgt tggaagagga acagcactgg ggagtctttg tggattctga   4148

ggccctgccc aatgagactc tagggtccag tggatgccac agcccagctt ggccctttcc   4208

ttccagatcc tgggtactga aagccttagg gaagctggcc tgagagggga agcggcccta   4268

agggagtgtc taagaacaaa agcgacccat tcagagactg tccctgaaac ctagtactgc   4328

cccccatgag gaaggaacag caatggtgtc agtatccagg ctttgtacag agtgcttttc   4388

tgtttagttt ttactttttt tgttttgttt ttttaaagac gaaataaaga cccaggggag   4448

aatgggtgtt gtatggggag gcaagtgtgg ggggtccttc tccacaccca ctttgtccat   4508

ttgcaaatat attttggaaa ac                                            4530

<210>  12
<211>  1255
<212>  PRT
<213>  Homo sapiens

<400>  12

Met Glu Leu Ala Ala Leu Cys Arg Trp Gly Leu Leu Leu Ala Leu Leu
1               5                   10                  15

Pro Pro Gly Ala Ala Ser Thr Gln Val Cys Thr Gly Thr Asp Met Lys
            20                  25                  30

Leu Arg Leu Pro Ala Ser Pro Glu Thr His Leu Asp Met Leu Arg His
        35                  40                  45

Leu Tyr Gln Gly Cys Gln Val Val Gln Gly Asn Leu Glu Leu Thr Tyr
    50                  55                  60

Leu Pro Thr Asn Ala Ser Leu Ser Phe Leu Gln Asp Ile Gln Glu Val
65                  70                  75                  80

Gln Gly Tyr Val Leu Ile Ala His Asn Gln Val Arg Gln Val Pro Leu
                85                  90                  95

Gln Arg Leu Arg Ile Val Arg Gly Thr Gln Leu Phe Glu Asp Asn Tyr
            100                 105                 110

Ala Leu Ala Val Leu Asp Asn Gly Asp Pro Leu Asn Asn Thr Thr Pro
        115                 120                 125

Val Thr Gly Ala Ser Pro Gly Gly Leu Arg Glu Leu Gln Leu Arg Ser
    130                 135                 140

Leu Thr Glu Ile Leu Lys Gly Gly Val Leu Ile Gln Arg Asn Pro Gln
145                 150                 155                 160

Leu Cys Tyr Gln Asp Thr Ile Leu Trp Lys Asp Ile Phe His Lys Asn
                165                 170                 175

Asn Gln Leu Ala Leu Thr Leu Ile Asp Thr Asn Arg Ser Arg Ala Cys
            180                 185                 190

His Pro Cys Ser Pro Met Cys Lys Gly Ser Arg Cys Trp Gly Glu Ser
        195                 200                 205

Ser Glu Asp Cys Gln Ser Leu Thr Arg Thr Val Cys Ala Gly Gly Cys
    210                 215                 220

Ala Arg Cys Lys Gly Pro Leu Pro Thr Asp Cys Cys His Glu Gln Cys
225                 230                 235                 240

Ala Ala Gly Cys Thr Gly Pro Lys His Ser Asp Cys Leu Ala Cys Leu
                245                 250                 255

His Phe Asn His Ser Gly Ile Cys Glu Leu His Cys Pro Ala Leu Val
            260                 265                 270

Thr Tyr Asn Thr Asp Thr Phe Glu Ser Met Pro Asn Pro Glu Gly Arg
        275                 280                 285

Tyr Thr Phe Gly Ala Ser Cys Val Thr Ala Cys Pro Tyr Asn Tyr Leu
    290                 295                 300

Ser Thr Asp Val Gly Ser Cys Thr Leu Val Cys Pro Leu His Asn Gln
305                 310                 315                 320

Glu Val Thr Ala Glu Asp Gly Thr Gln Arg Cys Glu Lys Cys Ser Lys
                325                 330                 335

Pro Cys Ala Arg Val Cys Tyr Gly Leu Gly Met Glu His Leu Arg Glu
            340                 345                 350

Val Arg Ala Val Thr Ser Ala Asn Ile Gln Glu Phe Ala Gly Cys Lys
        355                 360                 365

Lys Ile Phe Gly Ser Leu Ala Phe Leu Pro Glu Ser Phe Asp Gly Asp
    370                 375                 380

Pro Ala Ser Asn Thr Ala Pro Leu Gln Pro Glu Gln Leu Gln Val Phe
385                 390                 395                 400

Glu Thr Leu Glu Glu Ile Thr Gly Tyr Leu Tyr Ile Ser Ala Trp Pro
                405                 410                 415

Asp Ser Leu Pro Asp Leu Ser Val Phe Gln Asn Leu Gln Val Ile Arg
            420                 425                 430

Gly Arg Ile Leu His Asn Gly Ala Tyr Ser Leu Thr Leu Gln Gly Leu
        435                 440                 445

Gly Ile Ser Trp Leu Gly Leu Arg Ser Leu Arg Glu Leu Gly Ser Gly
    450                 455                 460

Leu Ala Leu Ile His His Asn Thr His Leu Cys Phe Val His Thr Val
465                 470                 475                 480

Pro Trp Asp Gln Leu Phe Arg Asn Pro His Gln Ala Leu Leu His Thr
                485                 490                 495

Ala Asn Arg Pro Glu Asp Glu Cys Val Gly Glu Gly Leu Ala Cys His
            500                 505                 510

Gln Leu Cys Ala Arg Gly His Cys Trp Gly Pro Gly Pro Thr Gln Cys
        515                 520                 525

Val Asn Cys Ser Gln Phe Leu Arg Gly Gln Glu Cys Val Glu Glu Cys
    530                 535                 540

Arg Val Leu Gln Gly Leu Pro Arg Glu Tyr Val Asn Ala Arg His Cys
545                 550                 555                 560

Leu Pro Cys His Pro Glu Cys Gln Pro Gln Asn Gly Ser Val Thr Cys
                565                 570                 575

Phe Gly Pro Glu Ala Asp Gln Cys Val Ala Cys Ala His Tyr Lys Asp
            580                 585                 590

Pro Pro Phe Cys Val Ala Arg Cys Pro Ser Gly Val Lys Pro Asp Leu
        595                 600                 605

Ser Tyr Met Pro Ile Trp Lys Phe Pro Asp Glu Glu Gly Ala Cys Gln
    610                 615                 620

Pro Cys Pro Ile Asn Cys Thr His Ser Cys Val Asp Leu Asp Asp Lys
625                 630                 635                 640

Gly Cys Pro Ala Glu Gln Arg Ala Ser Pro Leu Thr Ser Ile Val Ser
                645                 650                 655

Ala Val Val Gly Ile Leu Leu Val Val Val Leu Gly Val Val Phe Gly
            660                 665                 670

Ile Leu Ile Lys Arg Arg Gln Gln Lys Ile Arg Lys Tyr Thr Met Arg
        675                 680                 685

Arg Leu Leu Gln Glu Thr Glu Leu Val Glu Pro Leu Thr Pro Ser Gly

690                 695                 700

Ala Met Pro Asn Gln Ala Gln Met Arg Ile Leu Lys Glu Thr Glu Leu
705                 710                 715                 720

Arg Lys Val Lys Val Leu Gly Ser Gly Ala Phe Gly Thr Val Tyr Lys
                725                 730                 735

Gly Ile Trp Ile Pro Asp Gly Glu Asn Val Lys Ile Pro Val Ala Ile
            740                 745                 750

Lys Val Leu Arg Glu Asn Thr Ser Pro Lys Ala Asn Lys Glu Ile Leu
        755                 760                 765

Asp Glu Ala Tyr Val Met Ala Gly Val Gly Ser Pro Tyr Val Ser Arg
    770                 775                 780

Leu Leu Gly Ile Cys Leu Thr Ser Thr Val Gln Leu Val Thr Gln Leu
785                 790                 795                 800

Met Pro Tyr Gly Cys Leu Leu Asp His Val Arg Glu Asn Arg Gly Arg
                805                 810                 815

Leu Gly Ser Gln Asp Leu Leu Asn Trp Cys Met Gln Ile Ala Lys Gly
            820                 825                 830

Met Ser Tyr Leu Glu Asp Val Arg Leu Val His Arg Asp Leu Ala Ala
        835                 840                 845

Arg Asn Val Leu Val Lys Ser Pro Asn His Val Lys Ile Thr Asp Phe
    850                 855                 860

Gly Leu Ala Arg Leu Leu Asp Ile Asp Glu Thr Glu Tyr His Ala Asp
865                 870                 875                 880

Gly Gly Lys Val Pro Ile Lys Trp Met Ala Leu Glu Ser Ile Leu Arg
                885                 890                 895

Arg Arg Phe Thr His Gln Ser Asp Val Trp Ser Tyr Gly Val Thr Val
            900                 905                 910

Trp Glu Leu Met Thr Phe Gly Ala Lys Pro Tyr Asp Gly Ile Pro Ala
        915                 920                 925

Arg Glu Ile Pro Asp Leu Leu Glu Lys Gly Glu Arg Leu Pro Gln Pro
    930                 935                 940

Pro Ile Cys Thr Ile Asp Val Tyr Met Ile Met Val Lys Cys Trp Met
945                 950                 955                 960

Ile Asp Ser Glu Cys Arg Pro Arg Phe Arg Glu Leu Val Ser Glu Phe
                965                 970                 975

Ser Arg Met Ala Arg Asp Pro Gln Arg Phe Val Val Ile Gln Asn Glu
            980                 985                 990

Asp Leu Gly Pro Ala Ser Pro Leu  Asp Ser Thr Phe Tyr  Arg Ser Leu
        995                 1000                 1005

Leu Glu  Asp Asp Asp Met Gly  Asp Leu Val Asp Ala  Glu Glu Tyr
    1010                 1015                 1020

Leu Val  Pro Gln Gln Gly Phe  Phe Cys Pro Asp Pro  Ala Pro Gly
    1025                 1030                 1035

Ala Gly  Gly Met Val His His  Arg His Arg Ser Ser  Ser Thr Arg
    1040                 1045                 1050

Ser Gly  Gly Gly Asp Leu Thr  Leu Gly Leu Glu Pro  Ser Glu Glu
    1055                 1060                 1065

Glu Ala  Pro Arg Ser Pro Leu  Ala Pro Ser Glu Gly  Ala Gly Ser
    1070                 1075                 1080

Asp Val  Phe Asp Gly Asp Leu  Gly Met Gly Ala Ala  Lys Gly Leu
    1085                 1090                 1095

Gln Ser  Leu Pro Thr His Asp  Pro Ser Pro Leu Gln  Arg Tyr Ser
    1100                 1105                 1110

Glu Asp  Pro Thr Val Pro Leu  Pro Ser Glu Thr Asp  Gly Tyr Val
    1115                 1120                 1125

Ala Pro  Leu Thr Cys Ser Pro  Gln Pro Glu Tyr Val  Asn Gln Pro
    1130                 1135                 1140

Asp Val  Arg Pro Gln Pro Pro  Ser Pro Arg Glu Gly  Pro Leu Pro
    1145                 1150                 1155

Ala Ala  Arg Pro Ala Gly Ala  Thr Leu Glu Arg Ala  Lys Thr Leu
    1160                 1165                 1170

Ser Pro  Gly Lys Asn Gly Val  Val Lys Asp Val Phe  Ala Phe Gly
    1175                 1180                 1185

Gly Ala  Val Glu Asn Pro Glu  Tyr Leu Thr Pro Gln  Gly Gly Ala
    1190                 1195                 1200

Ala Pro  Gln Pro His Pro Pro  Pro Ala Phe Ser Pro  Ala Phe Asp
    1205                 1210                 1215

Asn Leu  Tyr Tyr Trp Asp Gln  Asp Pro Pro Glu Arg  Gly Ala Pro
    1220                 1225                 1230

Pro Ser Thr Phe Lys Gly Thr Pro Thr Ala Glu Asn Pro Glu Tyr
    1235                1240                1245

Leu Gly Leu Asp Val Pro Val
    1250                1255

<210> 13
<211> 18
<212> DNA
<213> Artificial sequence

<220>
<223> Synthetic primer, Her2 Forward primer1

<400> 13
tcctcctcgc cctcttgc                                                    18

<210> 14
<211> 18
<212> DNA
<213> Artificial sequence

<220>
<223> Synthetic primer, Her2 Forward primer 2

<400> 14
agccgcgagc acccaagt                                                    18

<210> 15
<211> 18
<212> DNA
<213> Artificial sequence

<220>
<223> Synthetic primer; Her2 Reverse primer 1

<400> 15
gcgggtctcc attgtcta                                                    18

<210> 16
<211> 21
<212> DNA
<213> Artificial sequence

<220>
<223> Synthetic primer; Her2 Reverse primer 2

<400> 16
ttggtgggca ggtaggtgag t                                                21

<210> 17
<211> 23
<212> DNA
<213> Artificial Sequence

<220>
<223> Synthetic primer; Cytokeratin 19 Forward primer

<400> 17
gcactacagc cactactaca cga                                              23

<210>  18
<211>  19
<212>  DNA
<213>  Artificial sequence

<220>
<223>  Synthetic primer; Cytokeratin 19 Reverse primer

<400>  18
ctcatgcgca gagcctgtt                                                    19


<210>  19
<211>  22
<212>  DNA
<213>  Artificial sequence

<220>
<223>  Synthetic primer; mammaglobin Forward primer 1

<400>  19
caatcaatcc acaagtgtct aa                                                22


<210>  20
<211>  22
<212>  DNA
<213>  Artificial sequence

<220>
<223>  Synthetic primer; mammaglobin; Forward primer 2

<400>  20
acggatgaaa ctctgagcaa tg                                                22


<210>  21
<211>  22
<212>  DNA
<213>  Artificial Sequence

<220>
<223>  Synthetic primer; mammaglobin; Reverse primer 1

<400>  21
aacatgtata gcaggtttca ac                                                22


<210>  22
<211>  20
<212>  DNA
<213>  Artificial sequence

<220>
<223>  Synthetic primer; Mammaglobin; Reverse primer 2

<400>  22
cagttctgtg agccaaaggt                                                   20